# FASCICULE DE BREVET EUROPEEN

(11) **EP 1 598 350 B1**
(45) Date de publication et mention de la délivrance du brevet: **11.07.2007**
(21) Numéro de dépôt: 05291066.8
(22) Date de dépôt: 18.05.2005
(51) Int. Cl.: C07D 405/12, C07D 409/12, C07D 401/12, C07D 417/12, A61K 31/4025, A61K 31/427, A61P 3/10

(54) **Dérivés de pyrrolidines et de thiazolidines, leur procédé de préparation et les utilisation pour le traitement de l'hyperglycémie et du diabète de type II**
Pyrrolidin- und thiazolidin-Derivate, deren Darstellung und Anwendung zur Behandlung von Hyperglykämie und Typ II Diabetes
Pyrrolidines and thiazolidines derivatives, their preparation and their use in the treatment of hyperglycaemia and of type II Diabetis

(30) Priorité: 19.05.2004 FR 0405454
(43) Date de publication de la demande: 23.11.2005
(73) Titulaire: Les Laboratoires Servier, 92415 Courbevoie Cédex (FR)
(72) Inventeur: De nanteuil, Guillaume, 92150 Suresnes (FR); Benoist, Alain, 95130 Franconville (FR); Combettes, Murielle, 78600 Maisons Laffite (FR); Harley, Elizabeth, 95490 Vaureal (FR)

(56) Documents cités:
- EP-A- 1 308 439
- WO-A2-01/96295
- WO-A2-98/19998
- WO-A2-03/057144
- WO-A2-03/074500
- US-A- 6 110 949
- DATABASE WPI Derwent Publications Ltd., London, GB; AN 2004-257167 XP002307917 -& WO 2004/016587 A (ONO PHARMACEUTICAL CO ; KONDO TAKASHI (JP); NAKAI HISAO (JP); YAMAMOTO) 26 février 2004 (2004-02-26)
- VILLHAUER E B ET AL: "1-ÄÄ(3-HYDROXY-1-ADAMANTYL)AMINOÜACETYLÜ- 2-CYANO-(S)-PYRROLIDINE: A POTENT, SELECTIVE, AND ORALLY BIOAVAILABLE DIPEPTIDYL PEPTIDASE IV INHIBITOR WITH ANTIHYPERGLYCEMIC PROPERTIES" JOURNAL OF MEDICINAL CHEMISTRY, AMERICAN CHEMICAL SOCIETY. WASHINGTON, US, vol. 46, no. 13, 2003, pages 2774-2789, XP001165747 ISSN: 0022-2623

## Description

La présente invention concerne de nouveaux dérivés de pyrrolidines et de thiazolidines, leur procédé de préparation, les compositions pharmaceutiques qui les contiennent ainsi que leur utilisation en tant qu'inhibiteurs de dipeptidyl-peptidase IV (DPP IV).

La dipeptidyl-peptidase IV est une sérine protéase membranaire, présente dans de nombreux tissus humains, et impliquée dans de nombreuses pathologies.

En particulier, il a été montré que la DPP IV était responsable de l'inactivation du GLP-1 (glucagon-like peptide-1). Or, le GLP-1 est un important stimulateur de la sécrétion d'insuline dans le pancréas et a donc un effet bénéfique direct sur le taux de glucose dans le sang.
L'inhibition de la DPP IV représente donc une approche extrêmement intéressante dans le traitement de l'intolérance au glucose et des maladies associées à l'hyperglycémie, telles que, par exemple, le diabète non insulino-dépendant (diabète de type II) ou l'obésité.

Des inhibiteurs de DPP IV ont déjà été décrits dans la littérature, notamment des dérivés d'amides dans la demande de brevet EP 1 308 439, dans la demande de brevet WO 2004/016587, dans le journal J. Med. Chem. 2003, 46 (13), 2774-89, dans la demande de brevet EP 0 490 379 et dans le journal Adv. Exp. Med. Biol. 1997, 421, 157-160, des dérivés de carbamates dans la demande de brevet DE 19826972, des dérivés d'α-amino-acides dans la demande de brevet EP 1 258 476 et des dérivés de sulfones dans la demande de brevet EP 1 245 568.

Les composés de l'invention possèdent des propriétés inhibitrices de dipeptidyl-peptidase IV, qui les rendent particulièrement utiles pour le traitement de l'intolérance au glucose et des maladies associées à l'hyperglycémie.

Plus spécifiquement, la présente invention concerne les composés de formule (I) : 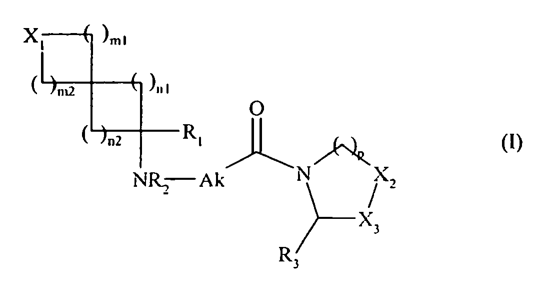 dans laquelle :
◆ X₁ représente un atome ou groupement choisi parmi CR₄ₐR_{4b}, O, S(O)_{q1} et N-R₅,
où R₄ₐ et R_{4b}, identiques ou différents, représentent chacun un atome d'hydrogène ou un groupement alkyle C₁-C₆ linéaire ou ramifié,
ou bien R₄ₐ et R_{4b} forment ensemble, avec l'atome de carbone qui les porte, un groupement cycloalkyle C₃-C₇,
q₁ représente zéro, 1 ou 2,
et R₅ représente un atome d'hydrogène ou un groupement alkyle C₁-C₆ linéaire ou ramifié, éventuellement substitué par un groupement hydroxy,
◆ m₁ représente zéro ou un entier compris entre 1 et 4 inclus,
◆ m₂ représente un entier compris entre 1 et 4 inclus,
◆ n₁ et n₂, identiques ou différents, représentent chacun un entier compris entre 1 et 3 inclus,
◆ R₁ représente un atome d'hydrogène ou un groupement choisi parmi carboxy, alkoxycarbonyle C₁-C₆ linéaire ou ramifié, carbamoyle éventuellement substitué par 1
ou 2 groupements alkyle C₁-C₆ linéaire ou ramifié, et alkyle C₁-C₆ linéaire ou ramifié éventuellement substitué par un groupement hydroxy ou amino éventuellement substitué par 1 ou 2 groupements alkyle C₁-C₆ linéaire ou ramifié,
◆ R₂ représente un atome d'hydrogène ou un groupement alkyle C₁-C₆ linéaire ou ramifié,
◆ Ak représente une chaîne alkylène C₁-C₄ linéaire ou ramifiée éventuellement substituée par un ou plusieurs atomes d'halogène, préférentiellement de fluor,
◆ p représente zéro, 1 ou 2,
◆ R₃ représente un atome d'hydrogène ou un groupement cyano,
◆ X₂ et X₃, identiques ou différents, représentent chacun, soit un groupement S(O)_{q2}, où q₂ représente zéro, 1 ou 2, soit un groupement CR₆ₐR_{6b}, où R₆ₐ et R_{6b}, identiques ou différents, représentent chacun un atome d'hydrogène ou d'halogène, préférentiellement de fluor, ou bien R₆ₐ représente un atome d'hydrogène et R_{6b} représente un groupement hydroxy,
leurs isomères optiques lorsqu'ils existent, ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, oxalique.

X₁ représente préférentiellement un atome d'oxygène ou un groupement -CH₂-.

m₁ et m₂ représentent chacun préférentiellement 1 ou 2.

n₁ et n₂ représentent chacun préférentiellement 1 ou 2, et sont préférentiellement identiques. 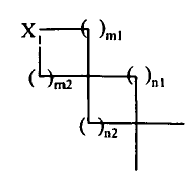 représente préférentiellement un groupement choisi parmi

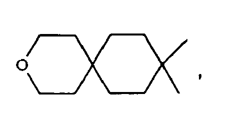

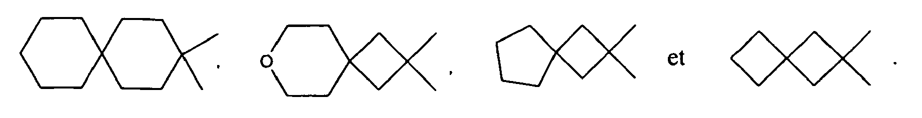

R₂ représente préférentiellement un atome d'hydrogène.

Ak représente préférentiellement un groupement -CH₂-.

p représente préférentiellement 1.

R₃ représente préférentiellement un groupement cyano. Dans ce cas, la configuration du carbone qui le porte est préférentiellement (S) lorsque X₂ et X₃ représentent chacun un groupement CR₆ₐR_{6b}, et (R) lorsque X₂ ou X₃ représente un groupement S(O)_{q2}.

X₂ et X₃ représentent chacun préférentiellement un groupement CR₆ₐR_{6b}.

Les composés préférés de formule (I) sont :
- le (2S)-1-({[9-(hydroxyméthyl)-3-oxaspiro[5.5]undéc-9-yl]-amino}-acétyl)-2-pyrrolidinecarbonitrile, ainsi que ses sels d'addition à un acide pharmaceutiquement acceptable;
- le (2S)-1-({[3-(hydroxyméthyl)spiro[5.5]undéc-3-yl]-amino}acétyl)-2-pyrrolidinecarbonitrile, ainsi que ses sels d'addition à un acide pharmaceutiquement acceptable;
- le (2S)-1-({[2-(hydroxyméthyl)-spiro[3.4]oct-2-yl]-amino}-acétyl)-2-pyrrolidinecarbonitrile, ainsi que ses sels d'addition à un acide pharmaceutiquement acceptable;
- le (4R)-3-[(spiro[5.5]undéc-3-yl-amino)-acétyl]-1,3-thiazolidine-4-carbonitrile, ainsi que ses sels d'addition à un acide pharmaceutiquement acceptable;
- le 2-({2-[(2S)-2-cyanopyrrolidinyl]-2-oxoéthyl}-amino)-spiro[3.3] heptane-2-carboxamide, ainsi que ses sels d'addition à un acide pharmaceutiquement acceptable;
- le (2S)-1-({[2-(hydroxyméthyl)-7-oxaspiro[3.5]non-2-yl]-amino}-acétyl)-2-pyrrolidinecarbonitrile, ainsi que ses sels d'addition à un acide pharmaceutiquement acceptable;
- le (2S, 4S)-4-fluoro-1-({[9-(hydroxyméthyl)-3-oxaspiro[5.5]undéc-9-yl]-amino}-acétyl)-2-pyrrolidinecarbonitrile, ainsi que son isomère (2S, 4R), son isomère (2R, 4R) et leurs sels d'addition à un acide pharmaceutiquement acceptable;
- le (2S)-4,4-difluoro-1-({[9-(hydroxyméthyl)-3-oxaspiro[5.5]undéc-9-yl]-amino}-acétyl)-2-pyrrolidinecarbonitrile, ainsi que ses sels d'addition à un acide pharmaceutiquement acceptable,
- et le (4R)-3-({[9-(hydroxyméthyl)-3-oxaspiro[5.5]undéc-9-yl]-amino}-acétyl)-1,3-thiazolidine-4-carbonitrile, ainsi que ses sels d'addition à un acide pharmaceutiquement acceptable.

L'invention s'étend également au procédé de préparation des composés de formule (I) à partir du composé de formule (II) : 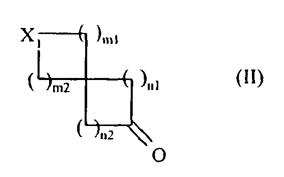 dans laquelle X₁, m₁, m₂, n₁ et n₂ sont tels que définis dans la formule (I),
que l'on transforme en composé de formule (III) : 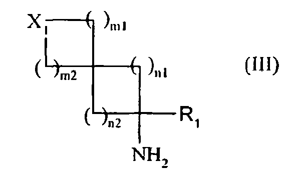 dans laquelle X₁, m₁, m₂, n₁, n₂ et R₁ sont tels que définis dans la formule (I),
que l'on met en réaction avec un composé de formule (IV) : 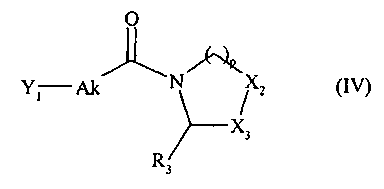 dans laquelle Ak, p, R₃, X₂ et X₃ sont tels que définis dans la formule (I)et Y₁ représente un groupement partant,
pour conduire aux composés de formule (Ia), cas particulier des composés de formule (I) pour lesquels R₂ représente un atome d'hydrogène : 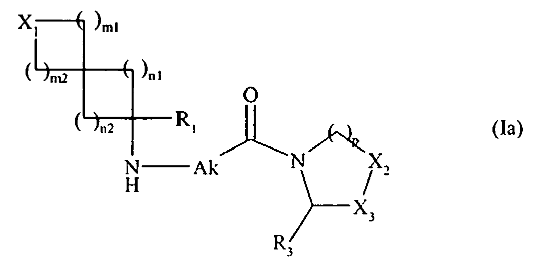 dans laquelle X₁, m₁, m₂, n₁, n₂, R₁, Ak, p, R₃, X₂ et X₃ sont tels que définis dans la formule (I),
que l'on met en réaction, lorsqu'on souhaite accéder aux composés de formule (I) pour lesquels R₂ est différent d'un atome d'hydrogène, avec un composé de formule (V) :

Y₂-R'₂ (V)

dans laquelle Y₂ représente un groupement partant, et R'₂ représente un groupement alkyle C₁-C₆ linéaire ou ramifié,
pour conduire aux composés de formule (Ib), cas particulier des composés de formule (I) pour lesquels R₂ représente un groupement alkyle C₁-C₆ linéaire ou ramifié : 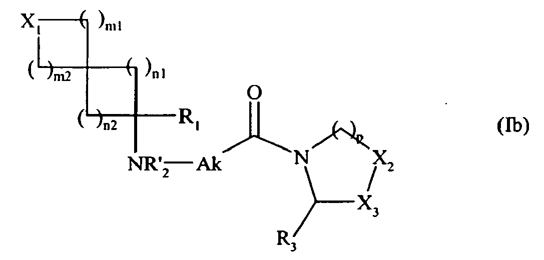 dans laquelle X₁, m₁, m₂, n₁, n₂, R₁, Ak, p, R₃, X₂ et X₃ sont tels que définis dans la formule (I), et R'₂ est tel que défini précédemment,
composés de formule (Ia) et (Ib) qui constituent l'ensemble des composés de formule (I), que l'on purifie, selon une technique classique de purification, dont on sépare éventuellement les isomères optiques selon une technique classique de séparation, et que l'on transforme éventuellement en leurs sels d'addition à un acide pharmaceutiquement acceptable.

Les composés de formule (IV) peuvent être préparés selon le procédé décrit dans J. Med. Chem. 2002, Vol 45(12), 2362-2365.

Lorsque l'on souhaite accéder aux composés de formule (I) pour lesquels R₁ représente un atome d'hydrogène, le composé de formule (II) est mis en réaction avec l'hydroxylamine, pour conduire au composé de formule (VI) : 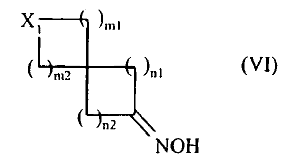 dans laquelle X₁, m₁, m₂, n₁ et n₂ sont tels que définis dans la formule (I),
qui est ensuite hydrogéné en composé de formule (IIIa), cas particulier des composés de formule (III) pour lesquels R₁ représente un atome d'hydrogène : 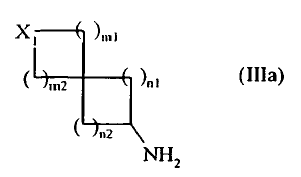 dans laquelle X₁, m₁, m₂, n₁ et n₂ sont tels que définis dans la formule (I).

Lorsque l'on souhaite accéder aux composés de formule (I) pour lesquels R₁ représente un groupement hydroxyméthyle, carboxy ou alkoxycarbonyle, le composé de formule (II) est mis en réaction avec du carbonate d'ammonium et du cyanure de potassium, pour conduire au composé de formule (VII) : 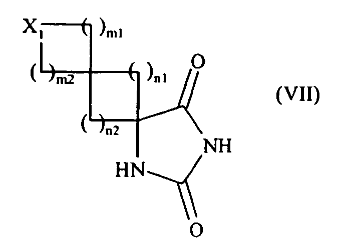 dans laquelle X₁, m₁, m₂, n₁ et n₂ sont tels que définis dans la formule (I),
qui est chauffé avec de la baryte, pour conduire au composé de formule (IIIb), cas particulier des composés de formule (III) pour lesquels R₁ représente un groupement carboxy : 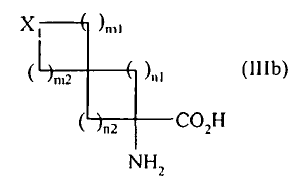 dans laquelle X₁, m₁, m₂, n₁ et n₂ sont tels que définis dans la formule (I),
qui est estérifié en composé de formule (IIIc), cas particulier des composés de formule (III) pour lesquels R₁ représente un groupement alkoxycarbonyle : 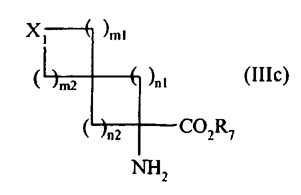 dans laquelle X₁, m₁, m₂, n₁ et n₂ sont tels que définis dans la formule (I), et R₇ représente un groupement alkyle C₁-C₆ linéaire ou ramifié,
dont la réduction conduit au composé de formule (IIId), cas particulier des composés de formule (III) pour lesquels R₁ représente un groupement hydroxyméthyle : 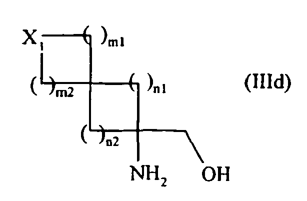 dans laquelle X₁, m₁, m₂, n₁ et n₂ sont tels que définis dans la formule (I).

Lorsque l'on souhaite accéder aux composés de formule (I) pour lesquels R₁ représente un groupement 1-hydroxy-1-méthyl-éthyle, le composé de formule (IIIc) est mis en réaction, après protection de la fonction amine, avec de l'iodure de méthylmagnésium, pour conduire, après déprotection de la fonction amine, au composé de formule (IIIe), cas particulier des composés de formule (III) pour lesquels R₁ représente un groupement 1-hydroxy-1-méthyl-éthyle : 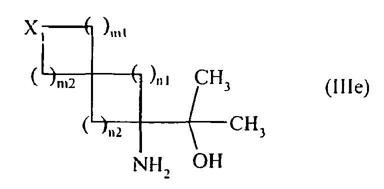 dans laquelle X₁, m₁, m₂, n₁ et n₂ sont tels que définis dans la formule (I).

Lorsque l'on souhaite accéder aux composés de formule (I) pour lesquels R₁ représente un groupement carbamoyle, le composé de formule (IIIb) est mis en réaction, après protection de la fonction amine, avec de l'ammoniac, en présence d'un agent de couplage, pour conduire, après déprotection de la fonction amine, au composé de formule (IIIf), cas particulier des composés de formule (III) pour lesquels R₁ représente un groupement carbamoyle : 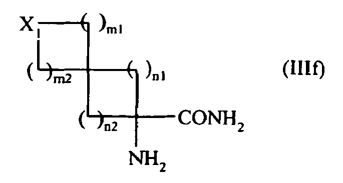 dans laquelle X₁, m₁, m₂, n₁ et n₂ sont tels que définis dans la formule (I).

Les composés de formule (II) peuvent être obtenus à partir du composé de formule (VIII) : 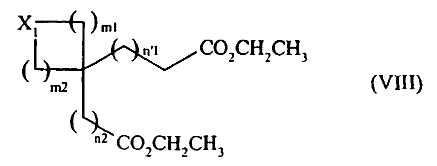 dans laquelle X₁, m₁, m₂ et n₂ sont tels que définis précédemment, et n'₁ représente zéro, 1 ou 2,
que l'on met en présence d'une base telle que l'hydrure de sodium, pour conduire au composé de formule (IX) : 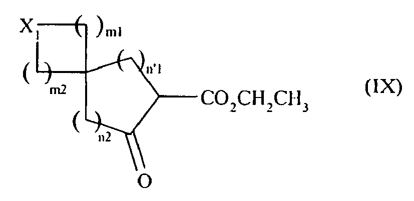 dans laquelle X₁, m₁, m₂, n'₁ et n₂ sont tels que définis précédemment,
dont la réaction avec de la potasse conduit au composé de formule (II).

Les composés de formule (lIa), cas particulier des composés de formule (II) pour lesquels n₁ et n₂ sont identiques et représentent chacun 1,
peuvent également être obtenus à partir du composé de formule (X) : 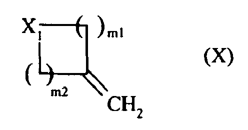 dans laquelle X₁, m₁ et m₂ sont tels que définis dans la formule (I),
qui est mis en réaction avec le chlorure de l'acide trichloroacétique et du zinc, pour conduire au composé de formule (XI) : 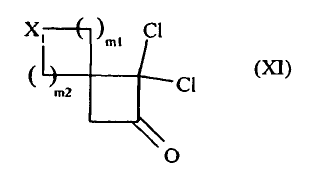 dans laquelle X₁, m₁ et m₂ sont tels que définis dans la formule (I),
dont la réduction conduit au composé de formule (IIa) : 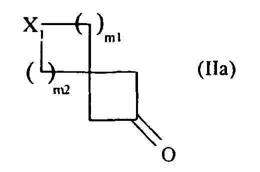 dans laquelle X₁, m₁ et m₂ sont tels que définis dans la formule (I).

Les composés de formule (IIb), cas particulier des composés de formule (II) pour lesquels n₁ et n₂ sont identiques et représentent chacun 2,
peuvent également être obtenus à partir du composé de formule (XII) : 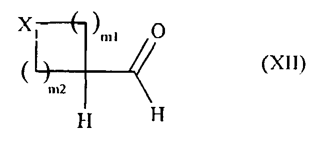 dans laquelle X₁, m₁ et m₂ sont tels que définis dans la formule (I),
que l'on met en réaction avec la méthylvinylcétone, pour conduire au composé de formule (XIII) : 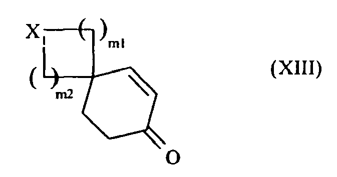 dans laquelle X₁, m₁ et m₂ sont tels que définis dans la formule (I),
dont l'hydrogénation catalytique conduit au composé de formule (IIb) : 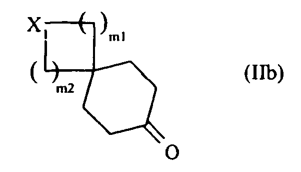 dans laquelle X₁, m₁ et m₂ sont tels que définis dans la formule (1).

Les composés de la présente invention, outre le fait qu'ils soient nouveaux, présentent des propriétés pharmacologiques intéressantes. Ils ont des propriétés inhibitrices de la dipeptidyl-peptidase IV qui les rendent utiles dans le traitement de l'intolérance au glucose et des maladies associées à une hyperglycémie telles que le diabète de type II ou l'obésité.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les préparations injectables, les suspensions buvables.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

Par composé de configuration (2RS), on entend mélange racémique des composés de configurations (2R) et (2S).

### EXEMPLE 1 : (2S)-1-({[9-(Hydroxyméthyl)-3-oxaspiro[5.5]undéc-9-yl]-amino}-acétyl)-2-pyrrolidinecarbonitrile :

### Stade A : 3-Oxaspiro[5.5]undéc-7-én-9-one

A 317 mmoles de tétrahydro-2H-pyranne-4-carboxaldéhyde et 317 mmoles de méthylvinylcétone dans 500 ml de benzène sont ajoutés 0,5 ml d'acide sulfurique. Le mélange réactionnel est ensuite porté à reflux pendant 3 heures en éliminant l'eau au moyen d'un appareil de Dean-Stark, puis 317 mmoles supplémentaires de méthylvinylcétone sont ajoutées, et le reflux est poursuivi 3 heures.
Le mélange est ensuite lavé, séché puis concentré, et le résidu obtenu est distillé pour conduire au produit attendu.

### Stade B : 3-Oxaspiro[5.5]undécan-9-one

147,4 mmoles du composé obtenu au stade précédent sont hydrogénés à température ambiante sous 4,6 bars pendant 15 heures, dans 100 ml d'acétate d'éthyle, en présence d'une quantité catalytique de Pd/C à 10%.
Le catalyseur est ensuite filtré, rincé à l'acétate d'éthyle et concentré, pour conduire au produit attendu.

### Stade C : 11-Oxa-1,3-diazadispiro[4.2.5.2]pentadécane-2,4-dione

A 300 mmoles du composé obtenu au stade précédent sont ajoutés 165 ml d'éthanol aqueux à 60% et 480 mmoles de carbonate d'ammonium. Le mélange réactionnel est ensuite chauffé à 55°C, puis 5,3 g de cyanure de potassium dans 40 ml d'eau sont additionnés en 5 mn, et le mélange est agité pendant 2 heures à 55°C. L'éthanol est ensuite évaporé, puis le mélange est filtré, le gâteau est rincé à l'eau et à l'acétone, puis séché, pour conduire au produit attendu sous la forme d'un solide floconneux blanc.

### Stade D : Acide 9-amino-3-oxaspiro[5.5]undécane-9 carboxylique, chlorhydrate

A 88,9 mmoles du composé obtenu au stade précédent dans 335 ml d'eau sont ajoutées 177,8 mmoles de baryte dans un autoclave de 1 litre. Le mélange est ensuite chauffé pendant la nuit à 160°C, puis refroidi dans un bain de glace. Le carbonate de baryum formé est filtré et rincé à l'eau, et du dioxyde de carbone est mis à buller dans la phase aqueuse.

La phase aqueuse est ensuite à nouveau filtrée, puis le filtrat est concentré à sec, pour conduire au produit attendu sous la forme d'une poudre.

### Stade E : 9-Amino-3-oxaspiro[5.5]undécane-9-carboxylate de méthyle, chlorhydrate

A 61,3 mmoles du composé obtenu au stade précédent sont ajoutés 100 ml de méthanol. La suspension obtenue est refroidie à 5°C, puis 184 mmoles de chlorure de thionyle sont additionnées au goutte à goutte. Le mélange réactionnel est ensuite agité 1 heure à 20°C, puis 2 heures à reflux, avant d'être évaporé à sec, pour conduire au produit attendu sous la forme d'une poudre.

### Stade F : (9-Amino-3-oxaspiro[5.5]undéc-9-yl)-méthanol

A 50 ml de tétrahydrofurane à 0°C sont ajoutés 6,8 g d'hydrure de lithium aluminium, puis 59,7 mmoles du composé obtenu au stade précédent sous forme de base en solution dans 75 ml de tétrahydrofurane. Le mélange réactionnel est ensuite agité pendant 30 mn à 0°C, puis pendant la nuit à 20°C.
Il est ensuite refroidi à 5°C, puis 6,8 ml d'eau, 6,8 ml de soude à 15% et 3 x 6,8 ml d'eau sont ajoutés. Le mélange est agité fortement pendant 20 mn, puis filtré sur Célite. Le précipité est rincé à l'éther et le filtrat est séché puis évaporé pour conduire au produit attendu sous la forme d'un solide.

### Stade G : (2S)-1-({[9-(Hydroxyméthyl)-3-oxaspiro[5.5]undéc-9-yl]-amino}-acétyl)-2-pyrrolidinecarbonitrile

A 12 mmoles du composé obtenu au stade précédent sont ajoutés 90 ml de dichlorométhane, 6 mmoles de (2S)-1-(chloroacétyl)-2-pyrrolidine carbonitrile puis 24 mmoles de carbonate de potassium. Après 6 jours d'agitation à 20°C, le précipité formé est filtré, rincé au dichlorométhane, et les filtrats sont concentrés à sec. Le résidu obtenu est purifié par chromatographie sur silice (éluant : dichlorométhane / méthanol 97/3) pour conduire au produit attendu sous la forme d'une huile jaune qui concrétise à froid pour donner une poudre blanche.

| *Microanalyse élémentaire :* | | | |
|---|---|---|---|
| | *% C* | *% H* | *% N* |
| *Calculé* | *64,45* | *8,71* | *12,53* |
| *Trouvé* | *64,50* | *8,78* | *12,28* |

### EXEMPLE 2 : (2S)-1-[(Spiro[5.5]undéc-3-yl-amino)-acétyl]-2-pyrrolidinecarbonitrile, chlorhydrate :

### Stade A : Spiro[5.5]undécan-3-one

Le produit attendu est obtenu selon le procédé décrit aux stades A et B de l'exemple 1, en remplaçant au stade A le tétrahydro-2H-pyranne-4-carboxaldéhyde par le cyclohexanecarboxaldéhyde.

### Stade B : Spiro[5.5]undécan-3-one oxime :

A 39 mmoles du composé obtenu au stade précédent en solution dans le dioxane sont ajoutés 70 ml de pyridine et 3,45 g de chlorhydrate d'hydroxylamine. Le mélange réactionnel est ensuite chauffé au reflux pendant la nuit, puis concentré à sec pour conduire à une huile, qui cristallise, et que l'on lave à l'eau, pour conduire après filtration et séchage au produit attendu sous la forme d'une poudre.

### Stade C: Spiro[5.5]undéc-3-yl amine

A 15,8 mmoles du composé obtenu au stade précédent en solution dans le dioxane sont ajoutés 3 ml d'ammoniaque concentrée et une quantité catalytique de nickel de Raney. Le mélange réactionnel est ensuite hydrogéné à pression et température ambiantes pendant la nuit. Le catalyseur est ensuite filtré, rincé, et la solution est concentrée, pour conduire au produit attendu sous la forme d'une huile.

### Stade D : (25)-1-[(Spiro[5.5]undéc-3-yl-amino)-acétyl]-2-pyrrolidinecarbonitrile

Le produit attendu est obtenu à partir du composé obtenu au stade précédent et de (2S)-1-(chloroacétyl)-2-pyrrolidinecarbonitrile, selon le procédé décrit au stade G de l'exemple 1.

### Stade E : (2S)-1-[(Spiro[5.5]undéc-3-yl-amino)-acétyl]-2-pyrrolidinecarbonitrile, chlorhydrate

Le produit attendu est obtenu par salification du composé obtenu au stade précédent à l'aide d'acide chlorhydrique.

| *Microanalyse élémentaire :* | | | | |
|---|---|---|---|---|
| | *% C* | *% H* | *% N* | *% Cl* |
| *Calculé* | *63,60* | *8,90* | *12,36* | *10,43* |
| *Trouvé* | *63,66* | *8,92* | *12,18* | *10,23* |

### EXEMPLE 3 :(2S)-1-[(Spiro[4.5]déc-8-yl-amino)-acétyl]-2-pyrrolidinecarbonitrile, chlorhydrate :

Le produit attendu est obtenu selon le procédé de l'exemple 2, en remplaçant, au stade A, le cyclohexanecarboxaldéhyde par le cyclopentanecarboxaldéhyde.

| *Microanalyse élémentaire :* | | | | |
|---|---|---|---|---|
| | *% C* | *% H* | *% N* | *% Cl* |
| *Calculé* | *62,66* | *8,66* | *12,89* | *10,88* |
| *Trouvé* | *62,58* | *8,76* | *12,92* | *10,97* |

### EXEMPLE 4 : N-[2-Oxo-2-(1-pyrrolidinyl)-éthyl]spiro[5.5]undéc-3-yl-amine, chlorhydrate :

Le produit attendu est obtenu selon le procédé de l'exemple 2, en remplaçant, au stade D, le (2S)-1-(chloroacétyl)-2-pyrrolidinecarbonitrile par la 1-(chloroacétyl)-pyrrolidine.

| *Microanalyse élémentaire :* | | | | |
|---|---|---|---|---|
| | *% C* | *% H* | *% N* | *% Cl* |
| *Calculé* | *64,84* | *9,92* | *8,90* | *11,26* |
| *Trouvé* | *65,54* | *9,89* | *8,98* | *11,28* |

### EXEMPLE 5 : (2S)-1-({[3-(Hydroxyméthyl)spiro[5.5]undéc-3-yl]-amino}acétyl)-2-pyrrolidinecarbonitrile :

Le produit attendu est obtenu selon le procédé de l'exemple 1, en remplaçant au stade A, le tétrahydro-2H-pyranne-4-carboxaldéhyde par le cyclohexanecarboxaldéhyde.

| *Microanalyse élémentaire :* | | | |
|---|---|---|---|
| | *% C* | *% H* | *% N* |
| *Calculé* | *68,43* | *9,37* | *12,60* |
| *Trouvé* | *68,78* | *9,32* | *12,45* |

### EXEMPLE 6 : (2S)-1-[(Spiro[5.7]tridéc-3-yl-amino)-acétyl]-2-pyrrolidinecarbonitrile, chlorhydrate :

Le produit attendu est obtenu selon le procédé de l'exemple 2, en remplaçant, au stade A, le cyclohexanecarboxaldéhyde par le cyclooctanecarboxaldéhyde.

| *Microanalyse élémentaire :* | | | | |
|---|---|---|---|---|
| | *% C* | *% H* | *% N* | *% Cl* |
| *Calculé* | *65,28* | *9,31* | *11,42* | *9,63* |
| *Trouvé* | *65,56* | *9,01* | *11,21* | *9,63* |

### EXEMPLE 7 : (2S)-1-[(3-Oxaspiro[5.5]undéc-9-yl-amino)-acétyl]-2-pyrrolidinecarbonitrile, chlorhydrate :

Le produit attendu est obtenu selon le procédé décrit aux stades B à E de l'exemple 2, à partir du composé obtenu au stade B de l'exemple 1.

| *Microanalyse élémentaire :* | | | | |
|---|---|---|---|---|
| | *% C* | *% H* | *% N* | *% Cl* |
| *Calculé* | *59,72* | *8,26* | *12,29* | *10,37* |
| *Trouvé* | *59,79* | *7,96* | *12,16* | *10,96* |

### EXEMPLE 8 : (2S)-1-[(Dispiro[5.2.5.2]hexadéc-3-yl-amino)-acétyl]-2-pyrrolidinecarbonitrile :

Le produit attendu est obtenu selon le procédé décrit aux stades A à D de l'exemple 2, en remplaçant, au stade A, le cyclohexanecarboxaldéhyde par le spiro[5.5]undécane-3-carboxaldéhyde.

| *Microanalyse élémentaire :* | | | |
|---|---|---|---|
| | *% C* | *% H* | *% N* |
| *Calculé* | *74,35* | *10,04* | *11,31* |
| *Trouvé* | *74,40* | *9, 94* | *11,07* |

### EXEMPLE 9 : (2S)-1-[(Spiro[3.3]hept-2-yl-amino)-acétyl]-2-pyrrolidinecarbonitrile, chlorhydrate :

### Stade A : 1,1-Dichlorospiro[3.3]heptan-2-one

A 5 g de méthylènecyclobutane en solution dans l'éther sont ajoutés 6,25 g de zinc en poudre, puis 11 ml de chlorure de l'acide trichloroacétique en solution dans 170 ml d'éther. La solution est soniquée en maintenant la température à 20°C pendant 3 heures, puis la solution noire est filtrée sur Whatman. Le filtrat est lavé, séché, filtré et concentré pour conduire au produit attendu sous la forme d'une huile brune.

### Stade B : Spiro[3.3]heptan-2-one

A 420 ml d'acide acétique glacial sont ajoutés 28 g du composé obtenu au stade précédent, puis 620 ml d'eau. Le milieu réactionnel est refroidi par un bain d'eau froide, puis 28,1 g de zinc en poudre sont ajoutés. Après 20 mn d'agitation, le bain est retiré et le mélange réactionnel est agité pendant la nuit à 20°C.
La solution est filtrée, extraite au pentane et évaporée. Le résidu est repris au pentane, et la solution est lavée, séchée, filtrée et concentrée pour conduire au produit attendu sous la forme d'un liquide jaune clair.

### Stade C : (2S)-1-[(Spiro[3.3]hept-2-yl-amino)-acétyl]-2-pyrrolidinecarbonitrile

Le produit attendu est obtenu selon le procédé décrit aux stades B à D de l'exemple 2, à partir du composé obtenu au stade précédent.

### Stade D : (2S)-1-[(Spiro[3.3]hept-2-yl-amino)-acétyl]-2-pyrrolidinecarbonitrile, chlorhydrate

Le produit attendu est obtenu par salification du composé obtenu au stade précédent à l'aide d'acide chlorhydrique.

| *Microanalyse élémentaire :* | | | | |
|---|---|---|---|---|
| | *% C* | *% H* | *% N* | *% Cl* |
| *Calculé* | *59,25* | *7,81* | *14,81* | *12,49* |
| *Trouvé* | *59,27* | *7,78* | *14,51* | *12,93* |

### EXEMPLE 10 : (2S)-1-(Spiro[5.9]pentadéc-3-yl-amino)-acétyl]-2-pyrrolidinecarbonitrile :

Le produit attendu est obtenu selon le procédé décrit aux stades A à D de l'exemple 2, en remplaçant au stade A le cyclohexanecarboxaldéhyde par le cyclodécanecarboxaldéhyde.
*Point de fusion* : 83°C

| *Microanalyse élémentaire :* | | | |
|---|---|---|---|
| | *%C* | *%H* | *%N* |
| *Calculé* | *73,49* | *10,37* | *11,69* |
| *Trouvé* | *73,46* | *10,44* | *11,64* |

### EXEMPLE 11 : (2S)-1-[(Spiro[3.5]non-7-yl-amino)-acétyl]-2-pyrrolidinecarbonitrile, chlorhydrate :

### Stade A : 7-Oxospiro[3.5]nonane-6-carboxylate d'éthyle

A 22,7 mmoles d'hydrure de sodium à 95 %, en suspension dans 15 ml de tétrahydrofurane, sont ajoutées à 0°C 20,6 mmoles d'ester éthylique de l'acide 3-[1-(éthoxycarbonyl-éthyl)-cyclobutyl]-propionique en solution dans le tétrahydrofurane. Le mélange réactionnel est chauffé à reflux pendant 4 heures, puis 49 ml d'acide acétique sont ajoutés. Le tétrahydrofurane est évaporé, et le résidu obtenu est extrait à l'acétate d'éthyle. Les phases organiques rassemblées sont lavées, filtrées, séchées et évaporées, pour conduire à une huile, qui est chromatographiée sur silice (éluant : dichlorométhane/acétate d'éthyle 95/5), pour conduire au produit attendu.

### Stade B : Spiro[3.5]nonan-7-one

A 39,7 mmoles du composé obtenu au stade précédent en solution dans le dioxane sont ajoutés 11,1 g de potasse dans 100 ml d'eau. Le mélange réactionnel est chauffé au reflux pendant une nuit, puis, après retour à température ambiante, il est extrait à l'éther. Les phases organiques rassemblées sont lavées, séchées, filtrées et évaporées, pour conduire au produit attendu sous la forme d'une huile volatile.

### Stade C : (2S)-1-[(Spiro[3.5]non-7-yl-amino)-acétyl]-2-pyrrolidinecarbonitrile, chlorhydrate

Le produit attendu est obtenu selon le procédé décrit aux stades B à E de l'exemple 2, à partir du composé obtenu au stade précédent.

| *Microanalyse élémentaire :* | | | | |
|---|---|---|---|---|
| | *% C* | *% H* | *% N* | *% Cl* |
| *Calculé* | *61,62* | *8,40* | *13,47* | *11,37* |
| *Trouvé* | *61,39* | *8,32* | *13,22* | *11,91* |

### EXEMPLE 12 : (2S)-1-[(Spiro[3.5)non-2-yl-amino)-acétyl)-2-pyrrolidinecarbonitrile, trifluoroacétate :

### Stade A : (2S)-1-[(Spiro[3.5]non-2-yl-amino)-acétyl]-2-pyrrolidinecarbonitrile

Le produit attendu est obtenu selon le procédé décrit aux stades A à C de l'exemple 9, en remplaçant, au stade A, le méthylènecyclobutane par le méthylènecyclohexane.

### Stade B : (2S)-1-[(Spiro[3.5]non-2-yl-amino)-acétyl]-2-pyrrolidinecarbonitrile, trifluoroacétate

Le produit attendu est obtenu par salification du composé obtenu au stade précédent à l'aide d'acide trifluoroacétique.
*Spectrométrie de masse : [M* + *H]*+ = *276*

### EXEMPLE 13 : (2S)-1-[(Spiro[5.6]dodéc-3-yl-amino)-acétyl]-2-pyrrolidinecarbonitrile :

Le produit attendu est obtenu selon le procédé décrit aux stades A à D de l'exemple 2, en remplaçant, au stade A, le cyclohexanecarboxaldéhyde par le cycloheptanecarboxaldéhyde.

| *Microanalyse élémentaire :* | | | |
|---|---|---|---|
| | *% C* | *% H* | *% N* |
| *Calculé* | *71,88* | *9,84* | *13,24* |
| *Trouvé* | *71,52* | *9,95* | *13,14* |

### EXEMPLE 14 : (2S)-1-[(Spiro[3.4]oct-2-yl-amino)-acétyl]-2-pyrrolidinecarbonitrile, chlorhydrate :

### Stade A : Spiro[3.4]octan-2-one

Le produit attendu est obtenu selon le procédé décrit aux stades A et B de l'exemple 9, en remplaçant, au stade A, le méthylènecyclobutane par le méthylènecyclopentane.

### Stade B : (2S)-1-[(Spiro[3.4]oct-2-yl-amino)-acétyl]-2-pyrrolidinecarbonitrile, chlorhydrate

Le produit attendu est obtenu selon le procédé décrit aux stades B à E de l'exemple 2, à partir du composé obtenu au stade précédent.

| *Microanalyse élémentaire :* | | | | |
|---|---|---|---|---|
| | *% C* | *% H* | *% N* | *% Cl* |
| *Calculé* | *60,49* | *8,12* | *14,11* | *11,90* |
| *Trouvé* | *61,08* | *8,00* | *14,18* | *11,37* |

### EXEMPLE 15 : (2S)-1-{[(3,3-Dioxo-3-thiaspiro[5.5]undéc-9-yl-amino)-acétyl]-2-pyrrolidinecarbonitrile :

Le produit attendu est obtenu selon le procédé décrit aux stades A à D de l'exemple 2, en remplaçant, au stade A, le cyclohexanecarboxaldéhyde par le tétrahydro-2*H*-thiopyranne-4-carboxaldéhyde 1,1-dioxyde.

| *Microanalyse élémentaire :* | | | | |
|---|---|---|---|---|
| | *% C* | *% H* | *% N* | *% S* |
| *Calculé* | *57,76* | *7,70* | *11,89* | *9,07* |
| *Trouvé* | *57,42* | *7,54* | *11,48* | *8,83* |

### EXEMPLE 16 : (2S)-1-({[2-(Hydroxyméthyl)-spiro[3.3]hept-2-yl]-amino}-acétyl)-2-pyrrolidinecarbonitrile, chlorhydrate :

### Stade A : (2S)-1-({[2-(Hydroxyméthyl)-spiro[3.3]hept-2-yl]-amino}-acétyl)-2-pyrrolidinecarbonitrile

Le produit attendu est obtenu selon le procédé décrit aux stades C à G de l'exemple 1, en remplaçant, au stade C, la 3-oxaspiro[5.5]undécan-9-one par le composé obtenu au stade B de l'exemple 9.

### Stade B : (2S)-1-({[2-(Hydroxyméthyl)-spiro[3.3]hept-2-yl]-amino}-acétyl)-2-pyrrolidinecarbonitrile, chlorhydrate

Le produit attendu est obtenu par salification du composé obtenu au stade précédent à l'aide d'acide chlorhydrique.

| *Microanalyse élémentaire :* | | | | |
|---|---|---|---|---|
| | *% C* | *% H* | *% N* | *% Cl* |
| *Calculé* | *57,41* | *7,71* | *13,39* | *11,30* |
| *Trouvé* | *57,32* | *8,02* | *13,33* | *11,91* |

### EXEMPLE 17 : (2S)-1-({[2-(Hydroxyméthyl)-spiro[3.4]oct-2-yl]-amino}-acétyl)-2-pyrrolidinecarbonitrile, chlorhydrate :

### Stade A : (2S)-1-({[2-(Hydroxyméthyl)-spiro[3.4]oct-2-yl]-amino}-acétyl)-2-pyrrolidinecarbonitrile

Le produit attendu est obtenu selon le procédé décrit aux stades C à G de l'exemple 1, en remplaçant, au stade C, la 3-oxaspiro[5.5]undécan-9-one par le composé obtenu au stade A de l'exemple 14.

### Stade B : (2S)-1-({[2-(Hydroxyméthyl)-spiro[3.4]oct-2yl]-amino}-acétyl)-2-pyrrolidinecarbonitrile, chlorhydrate

Le produit attendu est obtenu par salification du composé obtenu au stade précédent à l'aide d'acide chlorhydrique.

| *Microanalyse élémentaire :* | | | | |
|---|---|---|---|---|
| | *% C* | *% H* | *% N* | *% Cl* |
| *Calculé* | *58,62* | *7,99* | *12,82* | *10,81* |
| *Trouvé* | *58,53* | *7,90* | *12,65* | *10,81* |

### EXEMPLE 18 : (4R)-3-[(Spiro[5.5]undéc-3-yl-amino)-acétyl]-1,3-thiazolidine-4-carbonitrile, trifluoroacétate :

### Stade A : (4R)-3-[(Spiro[5.5]undéc-3-yl-amino)-acétyl]-1,3-thiazolidine-4-carbonitrile

Le produit attendu est obtenu selon le procédé décrit au stade G de l'exemple 1, à partir du composé obtenu au stade C de l'exemple 2 et de (4R)-3-(chloroacétyl)-1,3-thiazolidine-4-carbonitrile.

### Stade B : (4R)-3-[(Spiro[5.5]undéc-3-yl-amino)-acétyl]-1,3-thiazolidine-4-carbonitrile, trifluoroacétate

Le produit attendu est obtenu par salification du composé obtenu au stade précédent à l'aide d'acide trifluoroacétique.

| *Microanalyse élémentaire :* | | | | |
|---|---|---|---|---|
| | *% C* | *% H* | *% N* | *% S* |
| *Calculé* | *52,40* | *6,48* | *9,65* | *7,36* |
| *Trouvé* | *52,10* | *6,54* | *9,42* | *7,19* |

### EXEMPLE 19 : 2-({2-[(2S)-2-Cyanopyrrolidinyl]-2-oxoéthyl}-amino)-spiro[3.3] heptane-2-carboxamide :

### Stade A : Acide 2-aminospiro[3.3]heptane-2-carboxylique

Le produit attendu est obtenu selon le procédé décrit aux stades C et D de l'exemple 1, à partir du composé obtenu au stade B de l'exemple 9.

### Stade B : Acide 2-(tert-butyloxycarbonylamino)-spiro[3.3]heptane-2-carboxylique

A 4,35 g du composé obtenu au stade précédent en solution dans 11 ml de soude sont ajoutés 40 ml de dioxane, puis, à 0°C, 6,71 g de dicarbonate de di-(tert-butyle) dans le dioxane. Après une nuit à 20°C, le dioxane est évaporé et le résidu obtenu est repris à l'eau. On extrait à l'éther, puis acidifie la phase aqueuse à pH 3 au moyen d'une solution aqueuse à 10% d'acide citrique. La phase aqueuse est extraite à l'acétate d'éthyle. Les phases organiques sont réunies et lavées à la saumure, séchées et concentrées à sec pour conduire au produit attendu sous la forme d'une pâte blanche.

### Stade C : 2-(Tert-butyloxycarbonylamino)-spiro[3.3]heptane-2-carboxamide

A 1,8 g du composé obtenu au stade précédent en solution dans le tétrahydrofurane sont ajoutés 811 mg de N-hydroxysuccinimide et 1,45 g de dicyclohexylcarbodiimide. Après une nuit à 20°C, le mélange réactionnel est filtré, et on fait buller de l'ammoniac gazeux dans le filtrat. Après une nuit à 20°C, le mélange réactionnel est à nouveau filtré et concentré à sec, pour conduire au produit attendu sous la forme d'une poudre blanche.

### Stade D : 2-Aminospiro[3.3]heptane-2-carboxamide, chlorhydrate

1,84 g du composé obtenu au stade précédent sont mis en solution dans l'acétate d'éthyle, puis on fait buller à 0°C de l'acide chlorhydrique gazeux pendant 10 mn. Un précipité apparaît. Après 30 mn à 0°C, le précipité est filtré et rincé à l'acétate d'éthyle, puis séché, pour conduire au produit attendu sous la forme d'une poudre blanche.

### Stade E : 2-({2-[(2S)-2-Cyanopyrrolidinyl]-2-oxoéthyl}-amino)-spiro[3.3]heptane -2-carboxamide

Le produit attendu est obtenu selon le procédé décrit au stade G de l'exemple 1, à partir du composé obtenu au stade précédent et de (2S)-1-(chloroacétyl)-2-pyrrolidinecarbonitrile.

| *Microanalyse élémentaire :* | | | |
|---|---|---|---|
| | *% C* | *% H* | *% N* |
| *Calculé* | *62,05* | *7,64* | *19,30* |
| *Trouvé* | *62,05* | *7,48* | *19,20* |

### EXEMPLE 20 : (2S)-1-[(3-Azaspiro[5.5]undéc-9-yl-amino)-acétyl]-2-pyrrolidinecarbonitrile, bis (trifluoroacétate) :

### Stade A : 3-Azaspiro[5.5]undécan-9-one

Le produit attendu est obtenu selon le procédé décrit aux stades A et B de l'exemple 1, en remplaçant au stade A le tétrahydro-2H-pyranne-4-carboxaldéhyde par le 4-formyl-1-pipéridinecarboxylate de benzyle.

### Stade B : 9-Oxo-3-azaspiro[5.5]undécane-3-carboxylate de tert-butyle:

Le produit attendu est obtenu par réaction du composé obtenu au stade précédent avec du dicarbonate de di-tert-butyle, selon le procédé décrit au stade B de l'exemple 19.

### Stade C : 9-({2-[(2S)-2-Cyanopyrrolidinyl]-2-oxoéthyl}amino)-3-azaspiro[5.5]undécane-3-carboxylate de tert-butyle

Le produit attendu est obtenu selon le procédé décrit aux stades B à D de l'exemple 2, à partir du composé obtenu au stade précédent.

### Stade D : (2S)-1-[(3-Azaspiro[5.5]undéc-9-yl-amino)-acétyl]-2-pyrrolidinecarbonitrile, bis (trifluoroacétate)

Le produit attendu est obtenu par réaction du composé obtenu au stade précédent avec 30 équivalents d'acide trifluoroacétique dans le dichlorométhane à 0-5°C pendant 1 heure. Le milieu est concentré à sec. Le composé est purifié par HPLC préparative.
*Spectrométrie de masse : [M* + *H]* + = *305*

### EXEMPLE 21 : (2S)-1-({[3-Hydroxyméthyl)-9,9-diméthylspiro[5.5]undéc-3-yl]-amino}-acétyl)-2-pyrrolidinecarbonitrile :

Le produit attendu est obtenu selon le procédé de l'exemple 1, en remplaçant, au stade A, le tétrahydro-2H-pyranne-4-carboxaldéhyde par le 4,4-diméthyl-cyclohexane carboxaldéhyde.

| *Microanalyse élémentaire :* | | | |
|---|---|---|---|
| | *% C* | *% H* | *% N* |
| *Calculé* | *69,77* | *9,76* | *11,62* |
| *Trouvé* | *69,54* | *9,72* | *11,43* |

### EXEMPLE 22 : (2S)-({[2-(1-Hydroxy-1-méthyléthyl)-spiro[3.3]hept-2-yl]-amino}-acétyl)-2-pyrrolidinecarbonitrile, chlorhydrate :

### Stade A : 2-Aminospiro[3.3]heptane-2-carboxylate de méthyle

Le produit attendu est obtenu selon le procédé décrit au stade E de l'exemple 1, à partir du composé obtenu au stade A de l'exemple 19.

### Stade B : 2-(Tert-butyloxycarbonylamino)-spiro[3.3]heptane-2-carboxylate de méthyle

A 8,5 g du composé obtenu au stade précédent en solution dans le dichlorométhane sont ajoutés 5,8 ml de triéthylamine, puis, à 0°C, 9,02 g de dicarbonate de di-(tert-butyle) dans le dichlorométhane. Après une nuit d'agitation à 20°C, le milieu réactionnel est lavé avec une solution aqueuse à 10% d'acide citrique, puis à l'eau. La phase organique est séchée, filtrée et concentrée, pour conduire au produit attendu sous la forme d'une huile orange.

### Stade C : 2-(2-Tert-butyloxycarbonylamino-spiro[3.3]hept-2-yl)-2-propanol

A 50 ml d'éther sont ajoutés 42 ml d'une solution 3M d'iodure de méthylmagnésium dans l'éther, puis, à 0°C et au goutte à goutte, 5 g du composé obtenu au stade précédent en solution dans l'éther. Après 1 heure d'agitation à 0°C, du chlorure d'ammonium est ajouté, puis le mélange réactionnel est agité 1 heure à 20°C. La phase aqueuse est extraite à l'acétate d'éthyle, puis les phases organiques réunies sont lavées, séchées et concentrées à sec pour conduire au produit attendu sous la forme d'une huile qui cristallise.

### Stade D : 2-(2-Aminospiro[3.3]hept-2-yl)-2-propanol, trifluoroacétate

A 4,8 g du composé obtenu au stade précédent en solution dans le dichlorométhane sont ajoutés à 0°C 40 ml d'acide trifluoroacétique. Après 30 mn d'agitation à 0°C, le mélange réactionnel est évaporé à sec, le résidu est repris plusieurs fois au toluène, puis le toluène est évaporé, pour conduire au produit attendu sous la forme d'une huile incolore.

### Stade E : (2S)-1-({[2-(1-Hydroxy-1-méthyléthyl)-spiro[3.3]hept-2-yl]-amino}-acétyl)-2-pyrrolidinecarbonitrile

Le produit attendu est obtenu selon le procédé décrit au stade G de l'exemple 1, à partir du composé obtenu au stade précédent et de (2S)-1-(chloroacétyl)-2-pyrrolidinecarbonitrile.

### Stade F : (2S)-1-({[2-(1-Hydroxy-1-méthyléthyl)-spiro[3.3]hept-2yl]-amino}-acétyl)-2-pyrrolidinecarbonitrile, chlorhydrate

Le produit attendu est obtenu par salification du composé obtenu au stade précédent à l'aide d'acide chlorhydrique.

| *Microanalyse élémentaire :* | | | | |
|---|---|---|---|---|
| | *% C* | *% H* | *% N* | *% Cl* |
| *Calculé* | *59,72* | *8,26* | *12,29* | *10,37* |
| *Trouvé* | *58,96* | *8,48* | *11,95* | *10,99* |

### EXEMPLE 23 : (2S)-1-[(3-Thiaspiro[5.5]undéc-9-yl-amino)-acétyl]-2-pyrrolidinecarbonitrile, chlorhydrate :

### Stade A : 3-Thiaspiro[5.5]undécan-9-one oxime 3,3-dioxyde

Le produit attendu est obtenu selon le procédé décrit aux stades A et B de l'exemple 2, en remplaçant au stade A le cyclohexanecarboxaldéhyde par le tétrahydro-2H-thiopyranne-4-carboxaldéhyde 1,1-dioxyde.

### Stade B : 3-Thiaspiro[5.5]undécan-9-amine

4g du composé obtenu au stade précédent sont ajoutés par fractions à 7,9g d'hydrure de lithium aluminium en suspension dans 200ml de tétrahydrofurane. Le milieu réactionnel est ensuite porté à reflux pendant 12 heures, puis hydrolysé par addition de 8 ml d'eau, 8 ml d'une solution de soude à 15% et 16 ml d'eau.
Les sels obtenus sont ensuite filtrés, puis le filtrat est concentré à sec, pour conduire au produit du titre.

### Stade C : (2S)-1-[(3-Thiaspiro[5.5]undéc-9-yl-amino)-acétyl]-2-pyrrolidinecarbonitrile

Le produit attendu est obtenu selon le procédé décrit au stade G de l'exemple 1, à partir du composé obtenu au stade précédent et de (2S)-1-(chloroacétyl)-2-pyrrolidinecarbonitrile.

### Stade D: (2S)-J-[(3-Thiaspiro[5.5]undéc-9-yl-amino)-acétyl]-2-pyrrolidinecarbonitrile, chlorhydrate

Le produit attendu est obtenu par salification du composé obtenu au stade précédent à l'aide d'acide chlorhydrique.

| *Microanalyse élémentaire* : | | | | | |
|---|---|---|---|---|---|
| | *% C* | *% H* | *% N* | *% S* | *% Cl* |
| *Calculé* | *57,04* | *7,88* | *11,74* | *8,96* | *9,90* |
| *Trouvé* | *59,67* | *7,96* | *11,56* | *9,14* | *10,52* |

### EXEMPLE 24 : (2S)-1-({[3-(2-Hydroxyéthyl)-3-azaspiro[5.5]undéc-9-yl]-amino}-acétyl)-2-pyrrolidinecarbonitrile, bis (trifluoroacétate) :

### Stade A : 3-Azaspiro[5.5]undécan-9-one

Le produit attendu est obtenu selon le procédé décrit aux stades A et B de l'exemple 1, en remplaçant au stade A le tétrahydro-2H-pyranne-4-carboxaldéhyde par le 4-formyl-1-pipéridinecarboxylate de benzyle.

### Stade B : 3-(2-Hydroxyéthyl)-3-azaspiro[5.5]undécan-9-one

Le produit attendu est obtenu par alkylation du composé obtenu au stade précédent par le 2-bromoéthanol, en présence de carbonate de potassium.

### Stade C : (2S)-1-({[3-(2-Hydroxyéthyl)-3-azaspiro[5.5]undéc-9-yl]-amino}-acétyl)-2-pyrrolidinecarbonitrile

Le produit attendu est obtenu selon le procédé décrit aux stades B à D de l'exemple 2, à partir du composé obtenu au stade précédent.

### Stade D : (2S)-1-({[3-(2-Hydroxyéthyl)-3-azaspiro[5.5]undéc-9-yl]-amino}-acétyl)-2-pyrrolidinecarbonitrile, bis (trifluoroacétate)

Le produit attendu est obtenu par salification du composé obtenu au stade précédent à l'aide d'acide trifluoroacétique.

| *Microanalyse élémentaire :* | | | |
|---|---|---|---|
| | *% C* | *% H* | *% N* |
| *Calculé* | *47,92* | *5,94* | *9,72* |
| *Trouvé* | *47,28* | *5,94* | *9,38* |

### EXEMPLE 25 : (2S)-1-({[2-(Hydroxyméthyl)-7-oxaspiro[3.5]non-2-yl]-amino}-acétyl)-2-pyrrolidinecarbonitrile :

### Stade A : 7-Oxaspiro[3.5]nonan-2-one

Le produit attendu est obtenu selon le procédé décrit aux stades A et B de l'exemple 9, en remplaçant au stade A le méthylènecyclobutane par le 4-méthylènetétrahydro-2H-pyranne

### Stade B : (2S)-1-({[2-(Hydroxyméthyl)-7-oxaspiro[3.5]non-2-yl]-amino}-acétyl)-2-pyrrolidinecarbonitrile

Le produit attendu est obtenu selon le procédé décrit aux stades C à G de l'exemple 1, à partir du composé obtenu au stade précédent.
*Point de fusion : 103°C*

| *Microanalyse élémentaire :* | | | |
|---|---|---|---|
| | *% C* | *% H* | *% N* |
| *Calculé* | *62,52* | *8,20* | *13,67* |
| *Trouvé* | *62,56* | *8,25* | *13,32* |

### EXEMPLE 26 : (2S)1-({[2-(Hydroxyméthyl)-spiro[3.5]non-2yl]-amino}-acétyl)-2-pyrrolidinecarbonitrile:

### Stade A : Spiro[3.5]nonan-2-one

Le produit attendu est obtenu selon le procédé décrit aux stades A et B de l'exemple 9, en remplaçant au stade A le méthylènecyclobutane par le méthylènecyclohexane.

### Stade B : (2S)-1-({[2-(Hydroxyméthyl)-spiro[3.5]non-2-yl]-amino}-acétyl)-2-pyrrolidinecarbonitrile

Le produit attendu est obtenu selon le procédé décrit aux stades C à G de l'exemple 1, à partir du composé obtenu au stade précédent.

| *Microanalyse élémentaire :* | | | |
|---|---|---|---|
| | *% C* | *% H* | *% N* |
| *Calculé* | *66,85* | *8,91* | *13,76* |
| *Trouvé* | *66,66* | *8,55* | *13,65* |

### EXEMPLE 27 : (2S)-1-[(7-Oxaspiro[3,5]non-2-yl-amino)-acétyl]-2-pyrrolidinecarbonitrile, trifluoroacétate :

### Stade A : (2S)-1-[(7-Oxaspiro[3.5]non-2-yl-amino)-acétyl]-2-pyrrolidinecarbonitrile

Le produit attendu est obtenu selon le procédé décrit aux stades A à C de l'exemple 9, en remplaçant, au stade A, le méthylènecyclobutane par le 4-méthylènetétrahydro-2H-pyranne.

### Stade B : (2S)-1-[(7-Oxaspiro[3.5]non-2-yl-amino)-acétyl]-2-pyrrolidinecarbonitrile, trifluoroacétate

Le produit attendu est obtenu par salification du composé obtenu au stade précédent à l'aide d'acide trifluoroacétique.

| *Microanalyse élémentaire :* | | | |
|---|---|---|---|
| | %C | *%H* | *%N* |
| *Calculé* | *52,17* | *6,18* | *10,74* |
| *Trouvé* | *52,15* | *6,35* | *10,73* |

### EXEMPLE 28 : (2S)-1-[(Spiro[2.5]oct-6-yl-amino)-acétyl]-2-pyrrolidinecarbonitrile :

### Stade A : Spiro[2.5]octan-6-one

Le produit attendu est obtenu selon le procédé décrit aux stades A et B de l'exemple 11, en remplaçant, au stade A, l'acide 3-[1-(éthoxycarbonyl-éthyl)-cyclobutyl]-propionique par l'acide 3-[1-(éthoxycarbonyl-éthyl)-cyclopropyl]-propionique.

### Stade B : (2S)-1-[(Spiro[2.5]oct-6-yl-amino)-acétyl]-2-pyrrolidinecarbonitrile

Le produit attendu est obtenu selon le procédé décrit aux stades A à D de l'exemple 2, à partir du composé obtenu au stade précédent.

| *Microanalyse élémentaire :* | | | |
|---|---|---|---|
| | *% C* | *% H* | *% N* |
| *Calculé* | *68,93* | *8,87* | *16,08* |
| *Trouvé* | *68,90* | *8,76* | *16,06* |

### EXEMPLE 29 : (2S,4S)-4-Fluoro-1-({[9-(hydroxyméthyl)-3-oxaspiro[5.5]undéc-9-yl]-amino}-acétyl)-2-pyrrolidinecarbonitrile :

Le produit attendu est obtenu selon le procédé décrit dans l'exemple 1, en remplaçant, au stade G, le (2S)-1-(chloroacétyl)-2-pyrrolidinecarbonitrile par le (2S, 4S)-1-(chloroacétyl)-4-fluoro-2-pyrrolidinecarbonitrile.

| *Microanalyse élémentaire :* | | | |
|---|---|---|---|
| | %C | *%H* | *%N* |
| *Calculé* | *61,17* | *7,99* | *11,89* |
| *Trouvé* | *61,63* | *7,87* | *11,47* |

### EXEMPLE 30 : (2S)-4,4-Difluoro-1-({[9-(hydroxyméthyl)-3-oxaspiro[5.5]undéc-9-yl]-amino}-acétyl)-2-pyrrolidinecarbonitrile :

Le produit attendu est obtenu selon le procédé décrit dans l'exemple 1, en remplaçant, au stade G, le (2S)-1-(chloroacétyl)-2-pyrrolidinecarbonitrile par le (2S)-1-(chloroacétyl)-4,4-difluoro-2-pyrrolidinecarbonitrile.

| *Microanalyse élémentaire :* | | | |
|---|---|---|---|
| | *% C* | *% H* | *% N* |
| *Calculé* | *58,21* | *7,33* | *11,31* |
| *Trouvé* | *58,30* | *7,62* | *11,21* |

### EXEMPLE 31 : (9-{[2-Oxo-2-(1-pyrrolidinyl)-éthyl]-amino}-3-oxaspiro[5.5]undéc-9-yl)-méthanol :

Le produit attendu est obtenu selon le procédé décrit dans l'exemple 1, en remplaçant, au stade G, le (2S)-1-(chloroacétyl)-2-pyrrolidinecarbonitrile par la 1-(chloroacétyl)-pyrrolidine.

| *Microanalyse élémentaire :* | | | |
|---|---|---|---|
| | *% C* | *% H* | *% N* |
| *Calculé* | *65,77* | *9,74* | *9,02* |
| *Trouvé* | *65,75* | *9, 62* | *8,85* |

### EXEMPLE 32 : (4R)-3-({[9-(Hydroxyméthyl)-3-oxaspiro[5.5]undéc-9-yl]-amino}-acetyl)-1,3-thiazolidine-4-carbonitrile :

Le produit attendu est obtenu selon le procédé décrit dans l'exemple 1, en remplaçant, au stade G, le (2S)-1-(chloroacétyl)-2-pyrrolidinecarbonitrile par le (4R)-3-(chloroacétyl)-1,3-thiazolidine-4-carbonitrile.

| *Microanalyse élémentaire :* | | | | |
|---|---|---|---|---|
| | %C | *%H* | *%N* | %S |
| *Calculé* | *57,76* | *7,70* | *11,89* | *9,07* |
| *Trouvé* | *57,44* | *7,88* | *11,51* | *8,02* |

### EXEMPLE 33 : (2S,4S)-4-Hydroxy-1-({[9-(hydroxyméthyl)-3-oxaspiro[5.5]undéc-9-yl]-amino}-acétyl)-2-pyrrolidinecarbonitrile:

Le produit attendu est obtenu selon le procédé décrit dans l'exemple 1, en remplaçant, au stade G, le (2S)-1-(chloroacétyl)-2-pyrrolidinecarbonitrile par le (2S, 4S)-1-(chloroacétyl)-4-hydroxy-2-pyrrolidinecarbonitrile.

### EXEMPLE 34 : (2S,4R)-4-Fluoro-1-({[9-(hydroxyméthyl)-3-oxaspiro[5.5]undéc-9-yl]-amino}-acétyl)-2-pyrrolidinecarbonitrile, trifluoroacétate :

Le produit attendu est obtenu selon le procédé décrit dans l'exemple 1, en remplaçant, au stade G, le (2S)-1-(chloroacétyl)-2-pyrrolidinecarbonitrile par le (2S, 4R)-1-(chloroacétyl)-4-fluoro-2-pyrrolidinecarbonitrile.

| *Microanalyse élémentaire :* | | | |
|---|---|---|---|
| | %C | *%H* | *%N* |
| *Calculé* | *50,67* | *6,13* | *8, 77* |
| *Trouvé* | *50,47* | *6,05* | *8,47* |

### EXEMPLE 35 : [9-({2-[(3S)-3-Fluoropyrrolidinyl]-2-oxoéthyl}-amino)-3-oxaspiro [5.5]undéc-9-yl]méthanol :

Le produit attendu est obtenu selon le procédé décrit dans l'exemple 1, en remplaçant, au stade G, le (2S)-1-(chloroacétyl)-2-pyrrolidinecarbonitrile par la (3S)-1-(chloroacétyl)-3-fluoropyrrolidine.

### EXEMPLE 36 : (9-{[2-Oxo-2-(3,3,4,4-tétrafluoro-1-pyrrolidinyil-éthyl]-amino}-3-oxaspiro[5.5]undéc-9-yl)-méthanol:

Le produit attendu est obtenu selon le procédé décrit dans l'exemple 1, en remplaçant, au stade G, le (2S)-1-(chloroacétyl)-2-pyrrolidinecarbonitrile par la 1-(chloroacétyl)-3,3,4,4-tétrafluoropyrrolidine.

| *Microanalyse élémentaire :* | | | |
|---|---|---|---|
| | %C | *%H* | *%N* |
| *Calculé* | *53,40* | *6,85* | *7,33* |
| *Trouvé* | *53,83* | *6,67* | *7,18* |

### EXEMPLE 37 : (2S)-1-({[7-(Hydroxyméthyl)-spiro[3.5]non-7-yl]-amino}-acétyl)-2-pyrrolidinecarbonitrile :

Le produit attendu est obtenu selon le procédé décrit dans l'exemple 1, en remplaçant, au stade C, la 3-oxaspiro[5.5]undécan-9-one par le composé obtenu au stade B de l'exemple 11.

### EXEMPLE 38 : (2S)-1-({[8-(Hydroxyméthyl)-spiro[4.5]déc-8-yl]-amino}-acétyl)-2-pyrrolidinecarbonitrile :

Le produit attendu est obtenu selon le procédé de l'exemple 1, en remplaçant, au stade A, le tétrahydro-2H-pyranne-4-carboxaldéhyde par le cyclopentanecarboxaldéhyde.

### EXEMPLE 39 : (2S)-1-{[(2,2-Dioxo-2-thiaspiro[3.5]non-7-yl)-amino]-acétyl}-2-pyrrolidinecarbonitrile :

Le produit attendu est obtenu selon le procédé décrit aux stades A à D de l'exemple 2, en remplaçant, au stade A, le cyclohexanecarboxaldéhyde par le 3-thiétanecarboxaldéhyde 1,1-dioxyde.

### EXEMPLE 40 : (2S)-1-[3-(Spiro[5.5]undéc-3-yl-amino)-propanoyl]-2-pyrrolidinecarbonitrile, chlorhydrate :

### Stade A : (2S)-1-[3-(Spiro[5.5]undéc-3-yl-amino)-propanoyl]-2-pyrrolidinecarbonitrile

Le produit attendu est obtenu selon le procédé décrit au stade G de l'exemple 1, à partir du composé obtenu au stade C de l'exemple 2 et de (2S)-1-(3-chloropropionyl)-2-pyrrolidinecarbonitrile.

### Stade B : (2S)-1-[3-(Spiro[5.5]undéc-3-yl-amino)-propanoyl]-2-pyrrolidinecarbonitrile, chlorhydrate

Le produit attendu est obtenu par salification du composé obtenu au stade précédent à l'aide d'acide chlorhydrique.
*Spectrométrie de masse : [M + H]*+ = *318.*

### EXEMPLE 41 : (2S)-1-((2RS)-2-{[9-(Hydroxyméthyl)-3-oxaspiro[5.5]undéc-9-yl]amino}propanoyl)-2-pyrrolidinecarbonitrile, trifluoroacétate

Le produit attendu est obtenu selon le procédé de l'Exemple 1, en remplaçant au stade G le (2S)-1-(chloroacétyl)-2-pyrrolidine carbonitrile par le (2S)-1-[(2RS)-2-bromopropanoyl]-2-pyrrolidinecarbonitrile.
*Spectrométrie de masse : [M* + *H]*+ = *350.*

### EXEMPLE 42 : (2R)-1-({[19-(Hydroxyméthyl)-3-oxaspiro[5.5]undéc-9-yl]-amino)-acétyl)-2-pyrrolidinecarbonitrile :

Le produit attendu est obtenu selon le procédé de l'Exemple 1, en remplaçant au stade G le (2S)-1-(chloroacétyl)-2-pyrrolidine carbonitrile par le (2R)-1-(chloroacétyl)-2-pyrrolidine carbonitrile.

| *Microanalyse élémentaire :* | | | |
|---|---|---|---|
| | *% C* | *% H* | *% N* |
| *Calculé* | *64,45* | *8,71* | *12,53* |
| *Trouvé* | *64,04* | *8,75* | *12,21* |

### EXEMPLE 43 : (2RS)-4,4-Difluoro-1-({[9-(hydroxyméthyl)-3-oxaspiro[5.5]undéc-9-yl]amino}acétyl)-2-pyrrolidinecarbonitrile

Le produit attendu est obtenu selon le procédé de l'Exemple 1, en remplaçant au stade G le (2S)-1-(chloroacétyl)-2-pyrrolidine carbonitrile par le (2RS)-1-(chloroacétyl)-4,4-difluoro-2-pyrrolidinecarbonitrile.

| *Microanalyse élémentaire :* | | | |
|---|---|---|---|
| | *% C* | *% H* | *% N* |
| *Calculé* | *58,21* | *7,33* | *11,31* |
| *Trouvé* | *57,46* | *7,41* | *10,94* |

### EXEMPLE 44 : (2R)-4,4-Difluoro-1-({[9-(hydroxyméthyl)-3-oxaspiro[5.5]undéc-9-yl]amino}acétyl)-2-pyrrolidinecarbonitrile

Le produit attendu est obtenu par séparation par chromatographie HPLC préparative chirale du composé racémique de l'Exemple 43.
*Point de fusion : 128°C.*

### ETUDE PHARMACOLOGIOUE DES COMPOSES DE L'INVENTION

### EXEMPLE 45 : Inhibition de la dipeptidyl-peptidase IV dans le rein de porc

### Préparation de la fraction membranaire de rein de porc

Le tissu de rein de porc (large white, âgé de 2 à 3 mois) a été homogénéisé, 5g de tissu dans 15mL du tampon Tris (114mM, pH 7.8-8), puis centrifugé à 150000xg pendant 30min à 4°C. Le culot a été repris dans 15mL de tampon et re-centrifugé 150000xg pendant 30min à 4°C. Ce culot a été solubilisé sous agitation dans 15mL de tampon avec 60mM n-octyl-β-glucopyranoside pendant 30 min à température ambiante. Après centrifugation à 150000xg pendant 30min à 4°C, le surnageant est dialysé (MWCO 12-14000) contre 114mM Tris pH 7.8-8, puis aliquoté à -80°C.

### Mesure de l'activité dipeptidyl peptidase IV (DPPIV) :

L'activité enzymatique est mesurée par le clivage d'un substrat chromogène, le glycyl-prolyl-p-nitroanilide (Gly-Pro-pNA), donnant Gly-Pro et pNA (*p*-nitroaniline); cette dernière est détectée par absorbance à 405nm. L'activité est mesurée en l'absence (contrôle) ou en présence de 10µL des inhibiteurs, avec 10µL de la préparation de rein de porc (0.81mU, 1U = 1 µmol de pNA produit/min à 37°C). L'incubation est lancée par l'addition du substrat (250µL), mis en solution dans du tampon Tris (114mM) pH 7.8-8, pendant 60min à 37°C. Pour les produits solubilisés dans du diméthylsulfoxyde, la concentration finale de ce dernier n'a jamais été supérieure à 0.37%. Les résultats ont été exprimés en pourcentage du contrôle, et les IC₅₀ (doses efficaces pour l'inhibition à 50 % de l'activité contrôle) déterminés en utilisant une analyse non-linéaire, entre 0 et 100% (GraphPad Prism version 4.01 pour Windows). Chaque point est effectué en quadruplicate et chaque détermination d'IC₅₀ 1 à 5 fois.

Les résultats obtenus (moyenne géométrique des IC₅₀) pour les composés représentatifs de l'invention sont rassemblés dans le tableau suivant :

| **Composé** | **IC₅₀ DPP IV (nM)** |
|---|---|
| Exemple 1 | 76,1 |
| Exemple 5 | 68,5 |
| Exemple 6 | 27,2 |
| Exemple 8 | 23,0 |
| Exemple 17 | 43,6 |
| Exemple 18 | 3,5 |
| Exemple 20 | 73,1 |
| Exemple 23 | 27,0 |
| Exemple 28 | 171 |
| Exemple 29 | 31,6 |
| Exemple 30 | 20,5 |

Ces résultats montrent que les composés de l'invention sont de puissants inhibiteurs de la DPP IV.

### EXEMPLE 46 : Composition pharmaceutique

Formule de préparation pour 1000 comprimés dosés à 10 mg
Composé de l'exemple 1 10 g
Hydroxypropylcellulose 2 g
Amidon de blé 10 g
Lactose 100 g
Stéarate de magnésium 3 g
Talc 3 g

## Revendications

1. Composé de formule (I) : 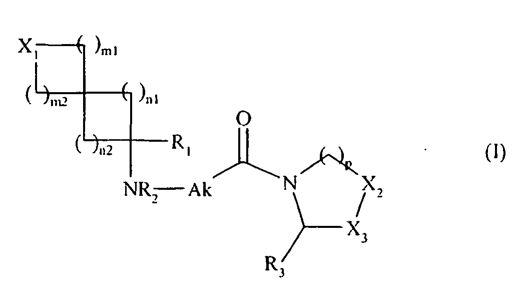 dans laquelle :
◆ X₁ représente un atome ou groupement choisi parmi CR₄ₐR_{4b}, O, S(O)_{q1} et NR₅, où R₄ₐ et R_{4b}, identiques ou différents, représentent chacun un atome d'hydrogène ou un groupement alkyle C₁-C₆ linéaire ou ramifié,
ou bien R₄ₐ et R_{4b} forment ensemble, avec l'atome de carbone qui les porte, un groupement cycloalkyle C₃-C₇,
q₁ représente zéro, 1 ou 2,
et R₅ représente un atome d'hydrogène ou un groupement alkyle C₁-C₆ linéaire ou ramifié, éventuellement substitué par un groupement hydroxy,
◆ m₁ représente zéro ou un entier compris entre 1 et 4 inclus,
◆ m₂ représente un entier compris entre 1 et 4 inclus,
◆ n₁ et n₂, identiques ou différents, représentent chacun un entier compris entre 1 et 3 inclus,
◆ R₁ représente un atome d'hydrogène ou un groupement choisi parmi carboxy, alkoxycarbonyle C₁-C₆ linéaire ou ramifié, carbamoyle éventuellement substitué par 1 ou 2 groupements alkyle C₁-C₆ linéaire ou ramifié, et alkyle C₁-C₆ linéaire ou ramifié éventuellement substitué par un groupement hydroxy ou amino éventuellement substitué par 1 ou 2 groupements alkyle C₁-C₆ linéaire ou ramifié,
◆ R₂ représente un atome d'hydrogène ou un groupement alkyle C₁-C₆ linéaire ou ramifié,
◆ Ak représente une chaîne alkylène C₁-C₄ linéaire ou ramifiée éventuellement substituée par un ou plusieurs atomes d'halogène, préférentiellement de fluor,
◆ p représente zéro, 1 ou 2,
◆ R₃ représente un atome d'hydrogène ou un groupement cyano,
◆ X₂ et X₃, identiques ou différents, représentent chacun, soit un groupement S(O)_{q2}, où q₂ représente zéro, 1 ou 2, soit un groupement CR₆ₐR_{6b}, où R₆ₐ et R_{6b}, identiques ou différents, représentent chacun un atome d'hydrogène ou d'halogène, préférentiellement de fluor, ou bien R₆ₐ représente un atome d'hydrogène et R_{6b} représente un groupement hydroxy,
ses isomères optiques lorsqu'ils existent, ainsi que ses sels d'addition à un acide pharmaceutiquement acceptable.

2. Composé de formule (I) selon la revendication 1, où X₁ représente un atome d'oxygène ou un groupement -CH₂-.

3. Composé de formule (I) selon l'une quelconque des revendications 1 ou 2, où m₁ et m₂ représentent chacun 1 ou 2.

4. Composé de formule (I) selon l'une quelconque des revendications 1 à 3, où n₁ et n₂ représentent chacun 1 ou 2, et sont identiques.

5. Composé de formule (I) selon la revendication 1, où 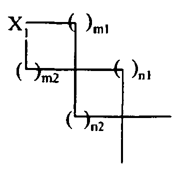 représente un groupement choisi parmi

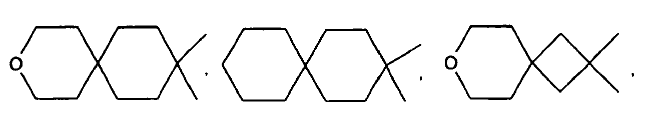

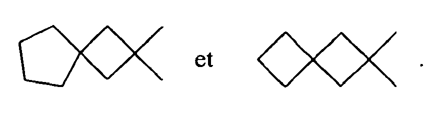

6. Composé de formule (I) selon l'une quelconque des revendications 1 à 5, où R₂ représente un atome d'hydrogène.

7. Composé de formule (I) selon l'une quelconque des revendications 1 à 6, où Ak représente un groupement -CH₂-.

8. Composé de formule (I) selon l'une quelconque des revendications 1 à 7, où p représente 1.

9. Composé de formule (I) selon l'une quelconque des revendications 1 à 8, où R₃ représente un groupement cyano.

10. Composé de formule (I) selon l'une quelconque des revendications 1 à 9, où X₂ représente un groupement CR₆ₐR_{6b}.

11. Composé de formule (I) selon l'une quelconque des revendications 1 à 10, où X₃ représente un groupement CR₆ₐR_{6b}.

12. Composé de formule (I) selon les revendications 9, 10 et 11, dont la configuration du carbone qui porte le groupement R₃ est (S).

13. Composé de formule (I) selon la revendication 9, où X₂ ou X₃ représente un groupement S(O)_{q2} et la configuration du carbone qui porte le groupement R₃ est (R).

14. Composé de formule (I) selon la revendication 1 qui est choisi parmi les composés suivants :
- le (2S)-1-({[9-(hydroxyméthyl)-3-oxaspiro[5.5]undéc-9-yl]-amino}-acétyl)-2-pyrrolidinecarbonitrile, ainsi que ses sels d'addition à un acide pharmaceutiquement acceptable;
- le (2S)-1-({[3-(hydroxyméthyl)spiro[5.5]undéc-3-yl]-amino}acétyl)-2-pyrrolidinecarbonitrile, ainsi que ses sels d'addition à un acide pharmaceutiquement acceptable;
- le (2S)-1-({[2-(hydroxyméthyl)-spiro[3.4]oct-2-yl]-amino}-acétyl)-2-pyrrolidinecarbonitrile, ainsi que ses sels d'addition à un acide pharmaceutiquement acceptable;
- le (4R)-3-[(spiro[5.5]undéc-3-yl-amino)-acétyl]-1,3-thiazolidine-4-carbonitrile, ainsi que ses sels d'addition à un acide pharmaceutiquement acceptable;
- le 2-({2-[(2S)-2-cyanopyrrolidinyl]-2-oxoéthyl}-amino)-spiro[3.3] heptane-2-carboxamide, ainsi que ses sels d'addition à un acide pharmaceutiquement acceptable;
- le (2S)-1-({[2-(hydroxyméthyl)-7-oxaspiro[3.5]non-2-yl]-amino}-acétyl)-2-pyrrolidinecarbonitrile, ainsi que ses sels d'addition à un acide pharmaceutiquement acceptable;
- le (2S,4S)-4-fluoro-1-({[9-(hydroxyméthyl)-3-oxaspiro[5.5]undéc-9-yl]-amino}-acétyl)-2-pyrrolidinecarbonitrile, ainsi que son isomère (2S, 4R), son isomère (2R, 4R) et leurs sels d'addition à un acide pharmaceutiquement acceptable;
- le (2S)-4,4-difluoro-1-({[9-(hydroxyméthyl)-3-oxaspiro[5.5]undéc-9-yl]-amino}-acétyl)-2-pyrrolidinecarbonitrile, ainsi que ses sels d'addition à un acide pharmaceutiquement acceptable,
- et le (4R)-3-({[9-(hydroxyméthyl)-3-oxaspiro[5.5]undéc-9-yl]-amino}-acétyl)-1,3-thiazolidine-4-carbonitrile, ainsi que ses sels d'addition à un acide pharmaceutiquement acceptable.

15. Procédé de synthèse des composés de formule (I) selon la revendication 1 à partir du composé de formule (II) : 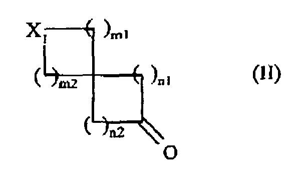 dans laquelle X₁, m₁, m₂, n₁ et n₂ sont tels que définis à la revendication 1,
que l'on transforme en composé de formule (III) : 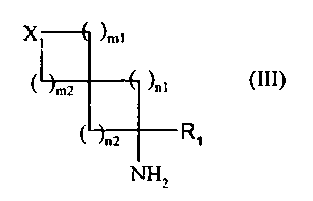 dans laquelle X₁, m₁, m₂, n₁, n₂ et R₁ sont tels que définis à la revendication 1,
que l'on met en réaction avec un composé de formule (IV) : 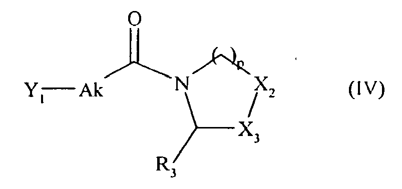 dans laquelle Ak, p, R₃, X₂ et X₃ sont tels que définis à la revendication 1, et Y₁ représente un groupement partant,
pour conduire aux composés de formule (la), cas particulier des composés de formule (I) pour lesquels R₂ représente un atome d'hydrogène : 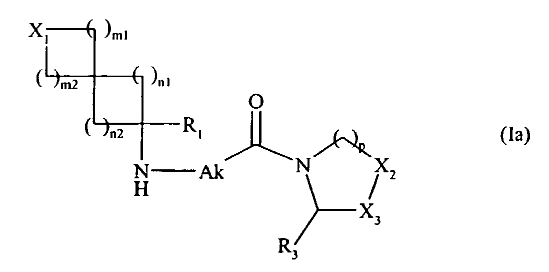 dans laquelle X₁, m₁, m₂, n₁, n₂, R₁, Ak, p, R₃, X₂ et X₃ sont tels que définis à la revendication 1,
que l'on met en réaction, lorsqu'on souhaite accéder aux composés de formule (I) pour lesquels R₂ est différent d'un atome d'hydrogène, avec un composé de formule (V) :
Y₂-R'₂ (V)
dans laquelle Y₂ représente un groupement partant, et R'₂ représente un groupement alkyle C₁-C₆ linéaire ou ramifié,
pour conduire aux composés de formule (Ib), cas particulier des composés de formule (I) pour lesquels R₂ représente un groupement alkyle C₁-C₆ linéaire ou ramifié : 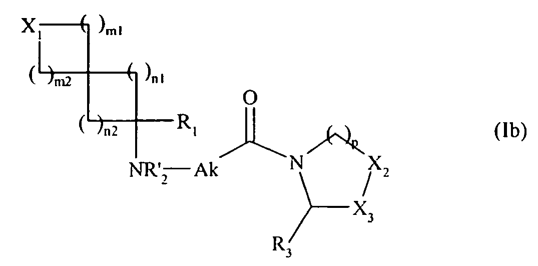 dans laquelle X₁, m₁, m₂, n₁, n₂, R₁, Ak, p, R₃, X₂ et X₃ sont tels que définis à la revendication 1, et R'₂ est tel que défini précédemment,
composés de formule (Ia) et (Ib) qui constituent l'ensemble des composés de formule (I),
que l'on purifie, selon une technique classique de purification, dont on sépare éventuellement les isomères optiques selon une technique classique de séparation, et que l'on transforme éventuellement en leurs sels d'addition à un acide pharmaceutiquement acceptable.

16. Composition pharmaceutique contenant comme principe actif un composé selon l'une quelconque des revendications 1 à 14, en combinaison avec un ou plusieurs véhicules inertes, non toxiques et pharmaceutiquement acceptables.

17. Utilisation d'un composé selon l'une quelconque des revendications 1 à 14 pour la fabrication de médicaments utiles comme inhibiteurs de dipeptidyl-peptidase IV.

18. Utilisation d'un composé selon l'une quelconque des revendications 1 à 14 pour la fabrication de médicaments utiles dans le traitement de l'intolérance au glucose et des maladies associées à une hyperglycémie.

19. Utilisation d'un composé selon l'une quelconque des revendications 1 à 14 pour la fabrication de médicaments utiles dans le traitement du diabète de type II.

## Claims

1. Compound of formula (I) : 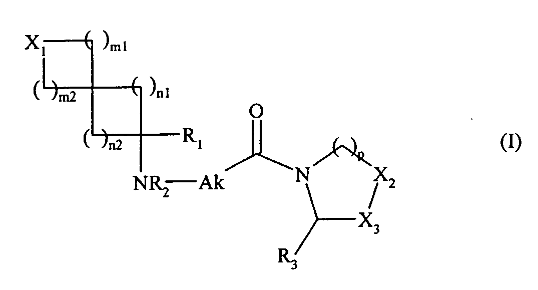 wherein :
◆ X₁ represents an atom or group selected from CR₄ₐR_{4b}, O, S(O)_{q1} and NR₅, wherein R₄ₐ and R_{4b}, which may be identical or different, each represent a hydrogen atom or a linear or branched C₁-C₆alkyl group,
or R₄ₐ and R_{4b} together form, with the carbon atom carrying them, a C₃-C₇cycloalkyl group,
q₁ represents zero, 1 or 2,
and R₅ represents a hydrogen atom or a linear or branched C₁-C₆alkyl group optionally substituted by a hydroxy group,
◆ m₁ represents zero or an integer of from 1 to 4 inclusive,
◆ m₂ represents an integer of from 1 to 4 inclusive,
◆ n₁ and n₂, which may be identical or different, each represent an integer of from 1 to 3 inclusive,
◆ R₁ represents a hydrogen atom or a group selected from carboxy, linear or branched C₁-C₆alkoxycarbonyl, carbamoyl optionally substituted by 1 or 2 linear or branched C₁-C₆alkyl groups, and linear or branched C₁-C₆alkyl optionally substituted by a hydroxy group or by an amino group optionally substituted by 1 or 2 linear or branched C₁-C₆alkyl groups,
◆ R₂ represents a hydrogen atom or a linear or branched C₁-C₆alkyl group,
◆ Ak represents a linear or branched C₁-C₄alkylene chain optionally substituted by one or more halogen, preferably fluorine, atoms,
◆ p represents zero, 1 or 2,
◆ R₃ represents a hydrogen atom or a cyano group,
◆ X₂ and X₃, which may be identical or different, each represent either a S(O)_{q2} group, wherein q₂ represents zero, 1 or 2, or a CR₆ₐR_{6b} group, wherein R₆ₐ and R_{6b}, which may be identical or different, each represent a hydrogen atom or a halogen, preferably fluorine, atom, or R₆ₐ represents a hydrogen atom and R_{6b} represents a hydroxy group,
its isomers, where they exist, and its addition salts with a pharmaceutically acceptable acid.

2. Compound of formula (I) according to claim 1, wherein X₁ represents an oxygen atom or a -CH₂- group.

3. Compound of formula (I) according to claim 1 or 2, wherein m₁ and m₂ each represent 1 or 2.

4. Compound of formula (I) according to any one of claims 1 to 3, wherein n₁ and n₂ each represent 1 or 2 and are identical.

5. Compound of formula (I) according to claim 1, wherein 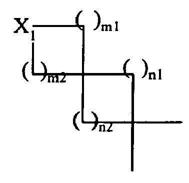 represents a group selected from

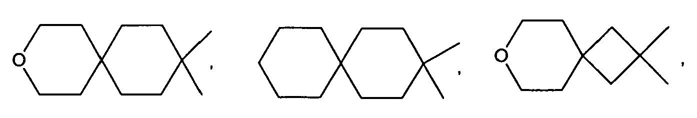

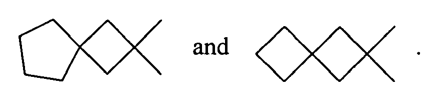

6. Compound of formula (I) according to any one of claims 1 to 5, wherein R₂ represents a hydrogen atom.

7. Compound of formula (I) according to any one of claims 1 to 6, wherein Ak represents a -CH₂- group.

8. Compound of formula (I) according to any one of claims 1 to 7, wherein p represents 1.

9. Compound of formula (I) according to any one of claims 1 to 8, wherein R₃ represents a cyano group.

10. Compound of formula (I) according to any one of claims 1 to 9, wherein X₂ represents a CR₆ₐR_{6b} group.

11. Compound of formula (I) according to any one of claims 1 to 10, wherein X₃ represents a CR₆ₐR_{6b} group.

12. Compound of formula (I) according to claims 9, 10 and 11, in which the configuration of the carbon carrying the R₃ group is (S).

13. Compound of formula (I) according to claim 9, wherein X₂ or X₃ represents a S(O)_{q2} group and the configuration of the carbon carrying the R₃ group is (R).

14. Compound of formula (I) according to claim 1 which is selected from the following compounds :
- (2S)-1-({[9-(hydroxymethyl)-3-oxaspiro[5.5]undec-9-yl]amino}acetyl)-2-pyrrolidinecarbonitrile, and its addition salts with a pharmaceutically acceptable acid;
- (2S)-1-({[3-(hydroxymethyl)spiro[5.5]undec-3-yl]amino}acetyl)-2-pyrrolidinecarbonitrile, and its addition salts with a pharmaceutically acceptable acid;
- (2S)-1-({[2-(hydroxymethyl)spiro[3.4]oct-2-yl]amino}acetyl)-2-pyrrolidinecarbonitrile, and its addition salts with a pharmaceutically acceptable acid;
- (4R)-3-[(spiro[5.5]undec-3-yl-amino)acetyl]-1,3-thiazolidine-4-carbonitrile, and its addition salts with a pharmaceutically acceptable acid;
- 2-({2-[(2S)-2-cyanopyrrolidinyl]-2-oxoethyl}amino)spiro[3.3]heptane-2-carboxamide, and its addition salts with a pharmaceutically acceptable acid;
- (2S)-1-({[2-(hydroxymethyl)-7-oxaspiro[3.5]non-2-yl]amino}acetyl)-2-pyrrolidinecarbonitrile, and its addition salts with a pharmaceutically acceptable acid;
- (2S,4S)-4-fluoro-1-({[9-(hydroxymethyl)-3-oxaspiro[5.5]undec-9-yl]amino}-acetyl)-2-pyrrolidinecarbonitrile, and its (2S,4R) isomer, its (2R,4R) isomer and addition salts thereof with a pharmaceutically acceptable acid;
- (2S)-4,4-difluoro-1-({[9-(hydroxymethyl)-3-oxaspiro[5.5]undec-9-yl]amino}-acetyl)-2-pyrrolidinecarbonitrile, and its addition salts with a pharmaceutically acceptable acid,
- and (4R)-3-({[9-(hydroxymethyl)-3-oxaspiro[5.5]undec-9-yl]amino}acetyl)-1,3-thiazolidine-4-carbonitrile, and its addition salts with a pharmaceutically acceptable acid.

15. Process for the synthesis of compounds of formula (I) according to claim 1 starting from a compound of formula (II) : 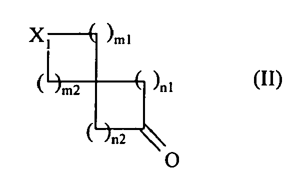 wherein X₁, m₁, m₂, n₁ and n₂ are as defined in claim 1,
which is converted to a compound of formula (III) : 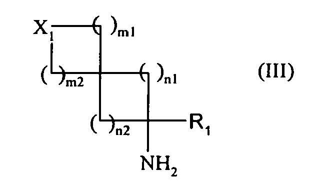 wherein X₁, m₁, m₂, n₁, n₂ and R₁ are as defined in claim 1,
which is reacted with a compound of formula (IV) : 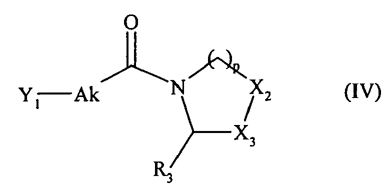 wherein Ak, p, R₃, X₂ and X₃ are as defined in claim 1 and Y₁ represents a leaving group,
to yield compounds of formula (Ia), a particular case of the compounds of formula (I) wherein R₂ represents a hydrogen atom : 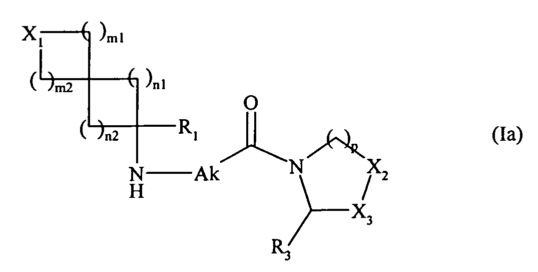 wherein X₁, m₁, m₂, n₁, n₂, R₁, Ak, p, R₃, X₂ and X₃ are as defined in claim 1,
which, when it is desired to obtain compounds of formula (I) wherein R₂ is other than a hydrogen atom, is reacted with a compound of formula (V) :
Y₂-R'₂ (V)
wherein Y₂ represents a leaving group and R'₂ represents a linear or branched C₁-C₆-alkyl group,
to yield compounds of formula (Ib), a particular case of the compounds of formula (I) wherein R₂ represents a linear or branched C₁-C₆alkyl group : 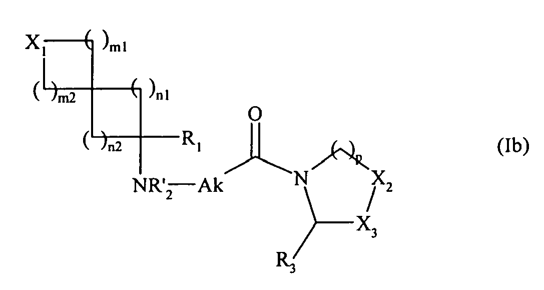 wherein X₁, m₁, m₂, n₁, n₂, R₁, Ak, p, R₃, X₂ and X₃ are as defined in claim 1 and R'₂ is as defined hereinbefore,
which compounds of formula (Ia) and (Ib), which constitute the totality of the compounds of formula (I),
are purified according to a conventional purification technique, are optionally separated into their optical isomers according to a conventional separation technique and, if desired, are converted into addition salts with a pharmaceutically acceptable acid.

16. Pharmaceutical composition comprising as active ingredient a compound according to any one of claims 1 to 14 in combination with one or more pharmaceutically acceptable, inert, non-toxic carriers.

17. Use of a compound according to any one of claims 1 to 14 for the manufacture of medicaments for use as dipeptidyl-peptidase IV inhibitors.

18. Use of a compound according to any one of claims 1 to 14 for the manufacture of medicaments for use in the treatment of glucose intolerance and of disorders associated with hyperglycaemia.

19. Use of a compound according to any one of claims 1 to 14 for the manufacture of medicaments for use in the treatment of type II diabetes.

## Patentansprüche

1. Verbindung der Formel (I): 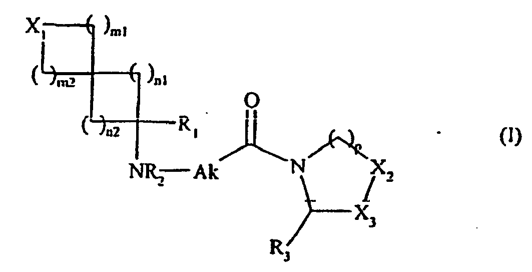 in der:
◆ X₁ ein Atom oder eine Gruppe bedeutet ausgewählt aus CR₄ₐR_{4b}, O, S(O)_{q1} und NR₅,
worin R₄ₐ und R_{4b}, die gleichartig oder verschieden sind, jeweils ein Wasserstoffatom oder eine geradkettige oder verzweigte C₁-C₆-Alkylgruppe bedeuten, oder R₄ₐ und R_{4b} gemeinsam mit dem sie tragenden Kohlenstoffatom eine C₃-C₇-Cycloalkylgruppe bilden,
q₁ Null, 1 oder 2 bedeutet und
R₅ ein Wasserstoffatom oder eine geradkettige oder verzweigte C₁-C₆-Alkylgruppe, die gegebenenfalls durch eine Hydroxygruppe substituiert ist, darstellt,
◆ m₁ Null oder eine ganze Zahl mit einem Wert zwischen 1 und 4 einschließlich bedeutet,
◆ m₂ eine ganze Zahl zwischen 1 und 4 einschließlich bedeutet,
◆ n₁ und n₂, die gleichartig oder verschieden sind, jeweils eine ganze Zahl mit einem Wert zwischen 1 und 3 einschließlich bedeuten,
◆ R₁ ein Wasserstoffatom oder eine Gruppe bedeutet ausgewählt aus Carboxy, geradkettigem oder verzweigtem C₁-C₆-Alkoxycarbonyl, Carbamoyl, gegebenenfalls substituiert durch 1 oder 2 geradkettige oder verzweigte C₁-C₆-Alkylgruppen, und geradkettigem oder verzweigtem C₁-C₆-Alkyl, gegebenenfalls substituiert durch eine Hydroxygruppe oder eine gegebenenfalls durch 1 oder 2 geradkettige oder verzweigte C₁-C₆-Alkylgruppen substituierte Aminogruppe,
◆ R₂ ein Wasserstoffatom oder eine geradkettige oder verzweigte C₁-C₆-Alkylgruppe bedeutet,
◆ Ak eine geradkettige oder verzweigte C₁-C₄-Alkylenkette bedeutet, die gegebenenfalls durch ein oder mehrere Halogenatome, vorzugsweise Fluoratome, substituiert ist,
◆ p Null, 1 oder 2 bedeutet,
◆ R₃ ein Wasserstoffatom oder eine Cyanogruppe bedeutet, und
◆ X₂ und X₃, die gleichartig oder verschieden sind, jeweils entweder eine Gruppe S(O)_{q2}, worin q₂ Null, 1 oder 2 darstellt, oder eine Gruppe CR₆ₐR_{6b} bedeuten, worin R₆ₐ und R_{6b}, die gleichartig oder verschieden sind, jeweils ein Wasserstoffatom oder ein Halogenatom, vorzugsweise ein Fluoratom, darstellen, oder R₆ₐ ein Wasserstoffatom und R_{6b} eine Hydroxygruppe bedeuten,
falls diese existieren, ihre optischen Isomeren sowie ihre Additionssalze mit einer pharmazeutisch annehmbaren Säure.

2. Verbindung der Formel (I) nach Anspruch 1, worin X₁ ein Sauerstoffatom oder eine Gruppe -CH₂- bedeutet.

3. Verbindung der Formel (I) nach einem der Ansprüche 1 oder 2, worin m₁ und m₂ jeweils 1 oder 2 bedeuten.

4. Verbindung der Formel (I) nach einem der Ansprüche 1 bis 3, worin n₁ und n₂ jeweils 1 oder 2 bedeuten und identisch sind.

5. Verbindung der Formel (I) nach Anspruch 1, worin 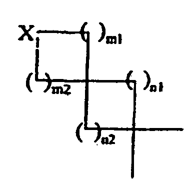 eine Gruppe bedeutet ausgewählt aus

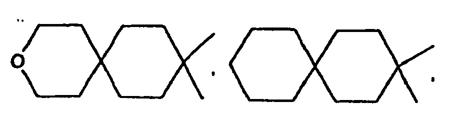

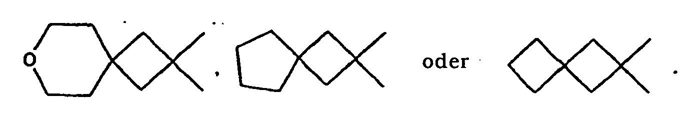

6. Verbindung der Formel (I) nach einem der Ansprüche 1 bis 5, worin R₂ ein Wasserstoffatom bedeutet.

7. Verbindung der Formel (I) nach einem der Ansprüche 1 bis 6, worin Ak eine Gruppe -CH₂- bedeutet.

8. Verbindung der Formel (I) nach einem der Ansprüche 1 bis 7, worin p 1 bedeutet.

9. Verbindung der Formel (I) nach einem der Ansprüche 1 bis 8, worin R₃ eine Cyanogruppe bedeutet.

10. Verbindung der Formel (I) nach einem der Ansprüche 1 bis 9, worin X₂ eine Gruppe CR₆ₐR_{6b} bedeutet.

11. Verbindung der Formel (I) nach einem der Ansprüche 1 bis 10, worin X₃ eine Gruppe CR₆ₐR_{6b} bedeutet.

12. Verbindung der Formel (I) nach den Ansprüchen 9, 10 und 11, bei denen die Konfiguration des die Gruppe R₃ tragenden Kohlenstoffatoms die Konfiguration (S) ist.

13. Verbindung der Formel (I) nach Anspruch 9, worin X₂ oder X₃ eine Gruppe S(O)_{q2} bedeutet und die Konfiguration des die Gruppe R₃ tragenden Kohlenstoffs die Konfiguration (R) ist.

14. Verbindung der Formel (I) nach Anspruch 1, ausgewählt aus den folgenden Verbindungen:
- (2S)-1-({[9-(Hydroxymethyl)-3-oxaspiro[5.5]undec-9-yl]-amino}-acetyl)-2-pyrrolidincarbonitril sowie dessen Additionssalze mit einer pharmazeutisch annehmbaren Säure;
- (2S)-1-({[3-(Hydroxymethyl)-spiro[5.5]undec-3-yl]-amino}-acetyl)-2-pyrrolidincarbonitril sowie dessen Additionssalze mit einer pharmazeutisch annehmbaren Säure;
- (2S)-1-({[2-(Hydroxymethyl)-spiro[3.4]oct-2-yl]-amino}-acetyl)-2-pyrrolidincarbonitril sowie dessen Additionssalze mit einer pharmazeutisch annehmbaren Säure;
- (4R)-3-[(Spiro[5.5]undec-3-yl-amino)-acetyl]-1,3-thiazolidin-4-carbonitril sowie dessen Additionssalze mit einer pharmazeutisch annehmbaren Säure;
- 2-({2-[(2S)-2-Cyanopyrrolidinyl]-2-oxoethyl}-amino)-spiro[3.3]heptan-2-carboxamid sowie dessen Additionssalze mit einer pharmazeutisch annehmbaren Säure;
- (2S)-1-({[2-(Hydroxymethyl)-7-oxaspiro[3.5]non-2-yl]-amino}-acetyl)-2-pyrrolidincarbonitril sowie dessen Additionssalze mit einer pharmazeutisch annehmbaren Säure;
- (2S, 4S)-4-Fluor-1-({[9-(hydroxymethyl)-3-oxaspiro[5.5]undec-9-yl]-amino}-acetyl)-2-pyrrolidincarbonitril sowie dessen Isomere (2S, 4R), dessen Isomere (2R, 4R) sowie dessen Additionssalze mit einer pharmazeutisch annehmbaren Säure;
- (2S)-4,4-Difluor-1-({[9-(hydroxymethyl)-3-oxaspiro[5.5]undec-9-yl]-amino}-acetyl)-2-pyrrolidincarbonitril sowie dessen Additionssalze mit einer pharmazeutisch annehmbaren Säure, und
- (4R)-3-({[9-(Hydroxymethyl)-3-oxaspiro[5.5]undec-9-yl]-amino}-acetyl)-1,3-thiazolidin-4-carbonitril sowie dessen Additionssalze mit einer pharmazeutisch annehmbaren Säure.

15. Verfahren zur Synthese der Verbindungen der Formel (I) nach Anspruch 1 ausgehend von der Verbindung der Formel (II): 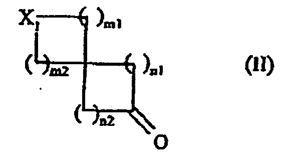 in der X₁, m₁, m₂, n₁ und n₂ die in Anspruch 1 angegebenen Bedeutungen besitzen, welche man in die Verbindungen der Formel (III) umwandelt: 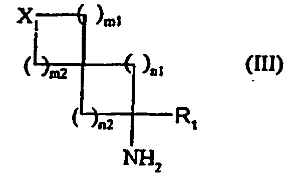 in der X₁, m₁, m₂, n₁, n₂ und R₁ die in Anspruch 1 angegebenen Bedeutungen besitzen,
welche man mit einer Verbindung der Formel (IV) umsetzt: 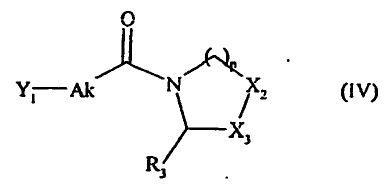 in der Ak, p, R₃, X₂ und X₃ die in Anspruch 1 angegebenen Bedeutungen besitzen und Y₁ eine austretende Gruppe darstellt,
zur Bildung der Verbindungen der Formel (Ia), einem Sonderfall der Verbindungen der Formel (I), worin R₂ ein Wasserstoffatom bedeutet: 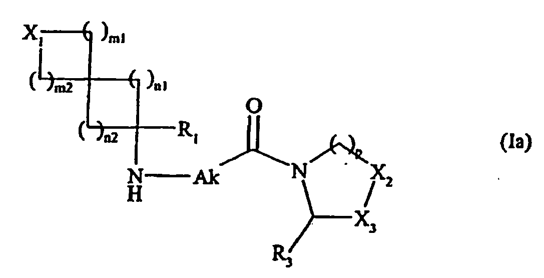 in der X₁, m₁, m₂, n₁, n₂, R₁, Ak, p, R₃, X₂ und X₃ die in Anspruch 1 angegebenen Bedeutungen besitzen,
welche man, wenn man die Verbindungen der Formel (I) erhalten möchte, worin R₂ von einem Wasserstoffatom verschieden ist, mit einer Verbindung der Formel (V) umsetzt:
Y₂ - R'₂ (V)
in der Y₂ eine austretende Gruppe und R'₂ eine geradkettige oder verzweigte C₁-C₆-Alkylgruppe bedeutet,
zur Bildung der Verbindungen der Formel (Ib), einem Sonderfall der Verbindungen der Formel (I), worin R₂ eine geradkettige oder verzweigte C₁-C₆-Alkylgruppe bedeutet: 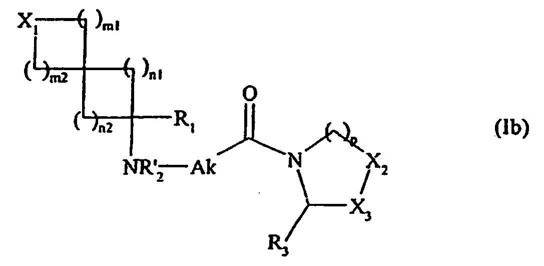 in der X₁, m₁, m₂, n₁, n₂, R₁, Ak, p, R₃, X₂ und X₃ die in Anspruch 1 angegebenen Bedeutungen besitzen und R'₂ die oben angegebenen Bedeutungen aufweist,
welche Verbindungen der Formeln (Ia) und (Ib), welche die Gesamtheit der Verbindungen der Formel (I) bilden, man mit Hilfe einer klassischen Reinigungsmethode reinigt, deren optische Isomeren man gegebenenfalls mit Hilfe einer klassischen Trennmethode trennt und die man gegebenenfalls in ihre Additionssalze mit einer pharmazeutisch annehmbaren Säure umwandelt.

16. Pharmazeutische Zubereitung enthaltend als Wirkstoff eine Verbindung nach einem der Ansprüche 1 bis 14 in Kombination mit einem oder mehreren inerten, nichttoxischen und pharmazeutisch annehmbaren Hilfsstoffen.

17. Verwendung einer Verbindung nach einem der Ansprüche 1 bis 14 für die Herstellung von Arzneimitteln, die als Inhibitoren der Dipeptidyl-peptidase IV nützlich sind.

18. Verwendung einer Verbindung nach einem der Ansprüche 1 bis 14 für die Herstellung von Arzneimitteln, die für die Behandlung von Glucoseintoleranz und mit Hyperglykämie verknüpften Erkrankungen geeignet sind.

19. Verwendung einer Verbindung nach einem der Ansprüche 1 bis 14 für die Herstellung von Arzneimitteln, die für die Behandlung von Diabetes Typ II nützlich sind.
